# EUROPEAN PATENT SPECIFICATION

(11) **EP 1 616 011 B1**
(45) Date of publication and mention of the grant of the patent: **03.10.2007**
(21) Application number: 04727873.4
(22) Date of filing: 16.04.2004
(51) Int. Cl.: C12N 15/62, C07K 14/035, C12N 9/16, G01N 33/50

(54) **CHIMERIC POLYPEPTIDES AND THEIR USE**
CHIMÄRE POLYPEPTIDE UND IHRE VERWENDUNG
POLYPEPTIDES CHIMERIQUES ET LEUR UTILISATION

(30) Priority: 18.04.2003 IT MI20030821
(43) Date of publication of application: 18.01.2006
(73) Proprietor: International Centre for Genetic Engineering and Biotechnology, 34012 Trieste (IT)
(72) Inventor: KÜHNE, Christian, A-1050 Vienna (AT); SIMONCSITS, Andras, 34012 Trieste (IT)
(74) Representative: Sonn & Partner Patentanwälte
(86) International application number: PCT/EP2004/004062
(87) International publication number: WO 2004/092194

(56) References cited:
- EP-A- 1 342 781
- WO-A-00/46386
- WO-A-00/58488
- WO-A-02/096952
- MELEKHOVETS Y F ET AL: "Fusion with an RNA binding domain to confer target RNA specificity to an RNase: design and engineering of Tat-RNase H that specifically recognizes and cleaves HIV-1 RNA in vitro" NUCLEIC ACIDS RESEARCH, OXFORD UNIVERSITY PRESS, SURREY, GB, vol. 24, no. 10, 1996, pages 1908-1912, XP002904225 ISSN: 0305-1048
- PHELAN A ET AL: "INTERCELLULAR DELIVERY OF FUNCTIONAL P53 BY THE HERPESVIRUS PROTEINVP22" NATURE BIOTECHNOLOGY, NATURE PUB. CO, NEW YORK, NY, US, vol. 16, no. 5, May 1998 (1998-05), pages 440-443, XP000979081 ISSN: 1087-0156
- "Voager Vectors; Voager Protein Production Kits" 2002, INVITROGEN CATALOGUE , XP002312634 pages 263-264
- MILLS KEVIN D ET AL: "MEC1-dependent redistribution of the Sir3 silencing protein from telomeres to DNA double-strand breaks" CELL, vol. 97, no. 5, 28 May 1999 (1999-05-28), pages 609-620, XP002312631 ISSN: 0092-8674
- MAIER F J ET AL: "MUTAGENESIS VIA INSERTIONAL - OR RESTRICTION ENZYME-MEDIATED - INTEGRATION (REMI) AS A TOOL TO TAG PATHOGENICITY RELATED GENES IN PLANT PATHOGENIC FUNGI" BIOLOGICAL CHEMISTRY, XX, XX, vol. 380, no. 7/8, July 1999 (1999-07), pages 855-864, XP009005657 ISSN: 1431-6730
- LUNDBERG M ET AL: "Is VP22 nuclear homing an artifact?" NATURE BIOTECHNOLOGY. AUG 2001, vol. 19, no. 8, August 2001 (2001-08), pages 713-714, XP002312632 ISSN: 1087-0156
- LUNDBERG MATHIAS ET AL: "Positively charged DNA-binding proteins cause apparent cell membrane translocation" BIOCHEMICAL AND BIOPHYSICAL RESEARCH COMMUNICATIONS, vol. 291, no. 2, 22 February 2002 (2002-02-22), pages 367-371, XP002312633 ISSN: 0006-291X
- KUHNE CHRISTIAN ET AL: "Repair of a minimal DNA double-strand break by NHEJ requires DNA-PKcs and is controlled by the ATM/ATR checkpoint." NUCLEIC ACIDS RESEARCH, vol. 31, no. 24, 15 December 2003 (2003-12-15), pages 7227-7237, XP002312878 ISSN: 0305-1048
- TANAKA M. ET AL: 'Gene therapy for mitochondrial disease by delivering restriction endonuclease SmaI into mitochondria.' J BIOMED SCI vol. 9, no. 6, 2002, pages 534 - 541
- DONAHUE S.L.: 'Mitochondrial DNA ligase function in Saccharomyces cerevisiae' NUCLEIC ACIDS RESEARCH vol. 29, no. 7, 2001, pages 1582 - 1589
- ROSENBERG ET AL: 'T7Select Phage Display System: A powerful new protein display system based on bacteriophage T7' INNOVATIONS no. 6, 1996, pages 1 - 6
- NISHIGORI ET AL: 'HindIII liposomes suppress delayed-type hypersensitivity responses in vivo and induce epidermal IL-10 in vitro.' THE JOURNAL OF IMMUNOLOGY vol. 161, 1998, pages 2684 - 2691

## Description

The technical field of the invention regards to molecules and methods for the study of the cellular systems that control DNA repair activity and the control of the cell cycle.

The genomes of living organisms are permanently exposed to chemical damage resulting from spontaneous endogenous chemical or biochemical damage or from exposition to exogenous genome damaging agents.

To ensure the necessary rates of genomic stability but also to provide the genomic flux essential for the evolution and the adaptation indispensable for all living species, highly complex systems for DNA-repair and DNA-recombination machineries have evolved in the cells. The equilibrium between the two systems responsible on one side for the fidelity of the genome and of the generation of molecular diversity is regulated by control mechanisms that ensure accuracy and precision. The levels of the controls are represented by the so called "checkpoint" or "surveillance control" that contribute to ensure the fidelity of the genome in any living organism. For a review about the mechanisms and the known genes involved and for a nomenclature of reverence see i.e. Zhou B. and S, Elledge, Nature, 2000, 408:433-439.

In recent years many molecular tools were developed for the analysis of the mechanism involved in DNA repair and more significant, for the control and regulation of these mechanisms. In US 6,307,015 for example, products and methods are described based on the protein Chk1, which are supposed to allow the identification of the mechanisms and gene products that are important for the control of the cellular checkpoints and DNA repair by the Chk1 protein.

Moreover, procedures for the introduction of recombination in the genome of a cell were developed: for an example Peitz et al. in Proc. Natl. Acad. Sci., 2002, 99: 4489-4494 obtained the translation of a chimeric Cre recombinase which is able to recombine lox-P sites that were previousely introduced into the genome with an efficiency in the range of 50%. Further, in wo 00/46386 the introduction of chromosomal recombination at specifically introduced SceI sites in combination with a vector that is expressing the meganuclease SceI is also described.

Tanaka et al. (J Biomed Sci 9 (2002) : 534-541) relates to a gene therapy for mitochondrial diseases by delivering the restriction endonuclease SmaI into mitochondria by providing a fusion protein comprising said restriction endonulcease fused to a mitochondrial target sequence.

In Donahue S.L. ((Nucleic Acid Res 29 (2001): 1582-1589) a fusion protein of a CDC9 mitochondrial targeting sequence fused to a restriction endonuclease is described. The gene encoding said fusion protein is introduced by genetic methods into the genome of Saccharomyces cerevisiae.

Rosenberg et al. (Innovations 6 (1996): 1-6) describe the phage display technology and discloses a phage nucleic acid molecule carrying a T7 endonuclease which when transfected to a host cell leads to the secretion and immobilisation of said endonuclease on the surface of said host.

Nishigori C. et al. (J Immunol 161 (1998) :2684-2691) relates to liposomes having encapsulated HindIII restriction endonucleases. Such liposomes may be used to provoke DNA damages in cells of the skin and, hence, to imitate changes caused regularly by UV-B radation.

In the WO00/58488 a method for modulating a cellular process by contacting a cell culture with a cell process modifying molecule conjugated to a translocating polypeptide is described. Said translocating protein is preferably the VP22-peptide of Herpes Simplex Virus type 1. The process modifying enzyme, which is fused to said translocating protein can be a DNA binding protein, in particular T7-RNA-polymerase.

The WO02/096952 relates to a protein conjugate which consists of two domains, wherein the first domain is a transport protein and the second domain a polypeptide for the regulation for a notch signal.

Phelan et al. (Nature Biotechn 16 (1998):440-443) describe a fusion protein comprising the Herpes Simplex virus protein VP22 fused to p53 protein which is present in a large number of tumors in a mutated form and is responsible for the regulation of the cell cycle.

Melekhovets et al. (Nucleic Acids Res 24 (1996):1908-1912) relates to fusion proteins which comprise a specific RNA/DNA binding and an RNA/DNA cleaving domain. The authors describe the production of a TAT (amino acids 1-72) as RNA binding domain in fusion to an RNAse H protein. This results in an RNAse H that has gained its function into an RNAse for the HIV-1 TAR RNA (RNAse H is naturally only working as an RNAse for the RNA part of a DNA-RNA hybrid as sole substrate) and it is the TAT 1-71 that provides specific RNA binding to TAR sequences.

The difficulty in the analysis of the repair of DNA damage after an induction with genotoxic agents is that all of these DNA damaging agents simultaneously cause a broad spectrum of different types of DNA lesions: thus the introduction of systems that act on DNA in a monospecific manner is of high interest. This would allow the definition of gene products with high precision that are involved in the cell cycle control pathways and in particular in DNA repair. The efficiency of these pathways is essential for a correct cellular replication, and their detailed understanding allows the development of screening systems for more selective pharmaceuticals and more directed therapeutic interventions.

An extensive range of somatic and hereditary disease is associated with defects in the DNA repair mechanism or in more general the control of the genetic information encoded by the DNA. Many of these defects are the prime cause for tumour pathology (for example mutations in the gene of p53. ARE', 14-3-3sigma, p16, Rb etc.), but moreover these defects could well be in addition responsible for many so far non characterized pathologies and that are simply classified as "somatic mutations" or "genetic predisposition". Already localized mutations in one of the pathways and that constitute the genetic base for hereditary disease are: Ataxia-telangiectasia (A-T), and the disorders that correlate with atassia (Ataxia-telangiectasia-like disorder ATLD, Mre11), the Nijmegen breakage syndrome (NBS), Fanconi anemia, Rothmund-Thomson syndrome, Non Hodgkin lymphomas (NHL), the Werner syndrome, the Blooms syndrome, the DNA Ligase IV (LIG4) syndrome, the Xeroderma pigmentosa, BRCAl.

DNA doublestrand breaks (DSB) represent the most dangerous damage of the genome. These can be induced for example by ionizing radiation, by reactive oxygen species (ROS) which can be either from exogenous sources but also from endogenous conditions for example by cellular stress. ROS are also induced from chemotherapeutical agents that are used in tumour therapy, for example by DNA intercalating or DNA crosslinking agents. The repair of DSBs is more difficult than other types of DNA damage: repair events and ligation of DSB ends can cause genetic instability due to loss, amplification or modification of the genetic material and are potentially tumourogenic.

Due to the fact that these events induce repair pathways and the cellular controls thereof, their understanding is of fundamental interest because this in turn can provide implications for a potential use in therapy.

The object of the present invention is therefore to provide an efficient agent for treating such defects in DNA repair mechanism or control of the genetic information. Preferably, these agents have to be delivered into the cells and especially into the nucleus to provide their activity there.

Therefore, the present invention provides a chimeric polypeptide comprising:
a. a polypeptide exhibiting affinity for specific nucleotide sequences
b. a DNA modifying enzyme
c. a region with intracellular delivery activity, wherein the DNA modifying enzyme is a restriction endonuclease from the type II class or their subunits or functional fragments and regions a., b., and c. are covalently linked to each other.

The present invention provides a system for the modification of the cellular genome by introducing DSBs with the chimeric molecules of the invention that exhibit specific cognate sites in the genome. One of the advantages of the chimeric molecules of the invention is the linear kinetic of the activity, the monospecific activity, and the fact that these molecules are capable to penetrate whole cell-populations also in a receptor independent mode. Advantageously these events caused by the chimeric molecules can be induced without selection by selective reagents and strongly simplifies their use.

The first embodiment of the invention relates to chimeric molecules that consist of: a region, preferred of polypeptide nature, that contains specific DNA binding activity, advantageously derived from a class II restriction enzyme; a region preferred of polypeptidic nature that exhibits a catalytic DNA modifying activity, consisting of an endonucleolytic activity from the type II class restriction enzymes, and a region with a cellular and/or nuclear membrane-crossing delivery activity. This said above functional regions are covalently linked amongst each other.

Another important embodiment of the invention relates to the isolated polynucleotides that code for the chimeric molecules of the invention if these are entirely or partially of polypeptide nature.

According to an other embodiment of the invention, that deduces from the activities of the polypeptides of the invention to interfere with key points of the cell cycle regulation and their checkpoint controls due to an introduction of DNA double strand breaks, the invention contains various procedures which are all characterized by the use of the chimeric molecules of the invention in cells in vivo. In particular these procedures according to the invention contain diagnostic procedures to evaluate genetic damage of the genes coding for the products that are involved in the control of the cell cycle and DNA repair including procedures for the selection of compositions with biological activities capable to modulate these control activities.

The invention also contains procedures that use the chimeric molecules of the invention to screen for new delivery activities or combinations of delivery activities.

The invention further provides for the therapeutic use of said compositions as anti-proliferatives, anti-neoplastic, antibiotic, antiparasitic or antiviral agents.

The various aspects of the invention are based on the results of the experiments provided by the inventors that it is possible to introduce monospecific DNA double strand breaks (DSB) into the genome of a cell in vivo and that these DSB's represent a sufficient signal for the activation of control points (check-points) during the cell cycle. Most of these activations are conserved throughout evolution from man to yeast and corresponding mechanisms are also present in prokaryotes and archaebacteria.

In eukaryotes these these activations are mediated by the modulation of the gene products from ATM (Ataxia-Teleangectasia Mutated protein), ATR (Ataxia-Teleangectasia Related), DNA-PKcs (DNA dependent Protein-Kinase - catalytic subunit), and their direct or indirect substrates such as Chk1 (Checkpoint-Kinase 1), Chk2 (Checkpoint-Kinase 2), Brca1 (Breast Cancer susceptibility-1), Brca2 (Breast Cancer susceptibility-2), Mre11 (Meiotic recombination 11), Rad50 (Radiation 50 double strand break repair), Nbs1 (Nijemegen breakage syndrome), Rad51 (Radiation 51 double strand break repair), FANCD2 (Fanconi anemia complementation D2), Histones, the helicases like BLM (Bloom's syndrome mutation), WRN (Werner's syndrome mutation), p53, and the direct or indirect transcriptional targets of p53 like for example p21 and 14-3-3 sigma, whereas this list is not complete and also many other targets are known and even yet to discover.

In particular it was found out that the activation of some of these gene-products is supposed to coordinate the maintain- ance of the homeostasis of the genome integrity during DNA repair, apoptosis or cell-cycle blocks.

The present invention provides chimeric molecules that are capable to cross the cellular membrane, to enter into the nucleus in the case of eukaryotic cells or into parasites in the cells, and to bind to specific sites in the DNA double strands, ideally modifying the DNA with first order kinetics.

Clearly, these findings and their mode of use opens the way for many practical applications for medicine as well as scientific purposes and applies for unique methods for the study of the mechanisms of DNA repair, control of DNA repair and of the cell cycle in general and for products capable to modify the genome of any cell in a selective fashion.

In a principal embodiment the invented chimeric molecules contain three functional components:
- a region, that consists of a polypeptide, that exhibits affinity for specific DNA or RNA sequences. This region is preferentially represented by a class II restriction enzyme, its subunits or functional fragments capable to recognize palindromic sequences in the DNA;
- a region, that consists of a polypeptide, exhibiting DNA endonucleolytic activity of a class II restriction enzyme;
- a region that contains a delivery-activity that is capable to cross the cellular membranes and/or nuclear membrane and transport heterologous molecules into the cell and into organelles; this activity is called "deliverer", whereas for "deliverer" is intended a chemical structure that preferred consists of a polypeptide or peptide capable to penetrate biological membranes of cells and for the case of eukaryotes also in addition the cellular membranes and the membranes of the nucleus or membranes of any other organelle including mitochondria or plastides. Specifically preferred are deliverers which enable transportation into the nucleus.

The functional domains outlined above are covalently connected. The chimeric molecules of the presented invention are obtained from chemical synthesis, or if the functional regions are of peptide nature, are synthesized preferentially by recombinant DNA technologies. For this embodiment and for the present invention the nucleotide sequences that code for the chimeric proteins containing the three functional regions as defined above, serve for the expression of the recombinant polypeptide in a host system, of preferred prokaryotic origin.

The chimerical molecules of the invention can also be produced with mixed techniques as well by chemical or recombinant methods, whereas at least one region is coupled with chemical methods to two other products that are produced by recombinant DNA techniques.

In a preferred embodiment in the chimerical molecule of the invention, the region with specific DNA binding activity is from a class II endonuclease. Preferentially chosen among the endonucleases: EcoRV, PvuII, HinfI, or their subunits or functional fragments. In the following of the presented invention as a functional fragment a polypeptide that includes as an amino acid sequence a sequence that is partially derived from the sequence of one of these entire proteins containing at least one function of the native enzyme is intended.

According to the present invention the region for the DNA modification activity is a class II restriction endonuclease and chosen preferably from the same endonuclease that contains the specific DNA binding activity and in particular: EcoRV, PvuII, HinfI or their functional subunits or fragments. In this preferred embodiment, in a single homodimeric protein the DNA recognition and DNA modification activities are connected.

In this last case, in a preferred embodiment, the restriction enzyme is advantageously contained as a single chain molecule, whereas all the subunits of the enzyme are covalently connected and expressed as a single chain polypeptide as is described for the enzyme PvuII in Simoncsits A. et al. J. Mol. scribed for the enzyme PvuII in Simoncsits A. et al. J. Mol. Biol, 2001, 309:89-97, maintaining the binding and cleavage characteristics comparable to the wild type enzyme.

The inventors have also developed in the frame of this invention a chimerical molecule where the restriction enzyme is contained as a single chain protein and where the modifying activity is changed or missing due to point mutations, deletions, or other structural variations of the region, subunit or catalytic domain of the restriction enzyme and the affinity for cognate DNA recognition and binding remains comparable to the native enzyme. Specifically preferred mutations include such mutants which have substantially retained (or even enhanced) their binding ability to the nucleic acid but have lost (significant part (e.g. > 50 %) or most of (>80 %)) their enzymatic (cutting) activity.

For this last aspect of the invention including also chimerical molecules that contain at least the region for transduction or intracellular delivery or deliverer and the DNA binding region as defined above and characterized by the fact that the amino acid sequence that exhibits the affinity for specific DNA sequences is preferred of endonucleolytic nature and has at least 95% sequence homology with a class II restriction enzyme. In such chimerical molecules the enzymatic activity is missing and they are used as vectors for a delivery to specific DNA sequences in the genome, without employing the capacity of modification. A preferred embodiment of this aspect of the invention is contained by the chimerical enzyme obtained by using instead of the native sequence of the PvuII enzyme (like in SCPVUTAT) the mutant D34G of the catalytic site obtained by the a substitution mutation in position 34 (Asp34/Gly34) of the enzyme PvuII as described in Nastri et al, 1997, J. Biol. Chem. 272:25761-25767. In such a mutated enzyme (SC34) the DNA recognizing activity is comparable to the wild type enzyme but the endonucleolytic activity is missing. Such molecules are also useful as controls to be used in parallel with the chimerical molecules that exhibit modification activity.

The deliverer or region for delivery across cellular membranes and/or nuclear membranes is advantageously a peptide. These is advantageously chosen among: the protein VP22 of HSV, the third alfa-helix of the homeodomain of tha Antennapedia protein, the proteins Tat and Rev of HIV-1 or their fragments or functional mutants. Examples for functional mutations are these described in Ho et al., 2001, Cancer Res., 61: 474-577. An preferred embodiment are the peptides from Tat and these that contain the functional domains with the sequence YGRKKRRQRRR (corresponds to region 47-57 of Tat) of the peptide SYGRKKR-RQRRRGGS. Functional mutations of this peptide amongst them the ones described in HO et al. are interchangeably used. In an other embodiment the deliverer sequences can be specific for any cell-type of any species and parasite including viruses. For example, sequences are chosen from bacterial deliverer sequences which leeds to a specific import of the chimeric molecules into prokaryotes. For illustration but no limitation bacterial deliverer can be choosen from oligopeptide sequences described and reviewed in Rajarao et al., 2002, FEMS Microbiology Letters; 215: 267-272. These peptides can contain a FKDE motif for a delivery across the membranes of E. coli like the sequence CFFKDEL and their functional derivatives. For example for S. aureus PFS containing motifs can be used such as VLTNENPFSDP and for B. subtilis the PFS containing motif YKKSNNPFSD. In another example molecules of the invention that contain sequences known in the art to penetrate into yeast such as homologs of the S. cerevisiae alpha and or a factors in combination with nuclear import sequences can be used for a yeast cell specific delivery. Examples of sequences for a delivery into various yeast strains are these described in Riezman et al., 1997; Cell 91, 731-738 and in Rajarao et al., 2002, FEMS Microbiology Letters; 215: 267-272, especially PFS-, YQR-, PFR-, PMF- or DCMD-containing motifs.

A preferred embodiment of this aspect of the invention is the use of classII restriction endonucleases that are able to cross bacterial membranes and to target and cleave their DNA. Restriction enzymes represent the most potent and highest developed naturally evolved killer system in the prokaryotic kingdom. The enzymatic nuclease activity resembles a vast amplification of a DNA damage activity and only traces of enzymes are needed to extinguish bacterial growth. In a preferred embodiment of this aspect of the invention these chimeric molecules can be used for antibiotic activities and can be advantagousely used to stop bacterial growth in a very selective way. Bacterial deliverers are supposed not to enter into other celltypes like human cells and in contrary deliverers like TAT sequences do not enter into bacterial cells.

Antibiotics, anti-tumour agents (e.g. cytostatics) and other further active agents may be specifically delivered by the agents according to the present invention, preferably by covalent coupling of the polypeptides of the present invention to such further active agents.

In a particular advantageous embodiment the deliverer or region for delivery across cellular membranes and/or nuclear and organelle membranes can contain an additional modifying component or "auxiliary" domain preferred a polypeptide or chemical compound. This domain can be connected with the deliverer sequences or can be placed in any other part of the chimeric molecules of the invention. The addition of these molecules can be obtained with techniques known in the art, such as recombinant techniques or chemical coupling. For illustration but not exclusion, in order to render the chimeric molecules of the invention cell type specific an additional auxiliary domain, that caries a binding site for cell-type specific and for example, tumour specific amplified receptors. These are for example the Her2, TGFß RI, or CD20 receptor. This can be obtained by adding the binding fragments of EGF, and TGFß or Fv or single chain Fv (scFv) immunoglobulin fragments into the auxiliary domain. In a particular embodiment the restriction enzyme is used in its native dimeric form, whereas to the N terminus of the first protein a specific light chain immunoglobulin fragment including functional CDRL regions is ligated and to the N terminus of the other protein of the homodimer the variable heavy chain immunoglobulin protein including functional CDR1-3H regions are linked. This chimeric proteins are able to dimerize by light chain heavy chain dimerisation and by dimerisation of the restriction enzyme domain. Thus, this can target specific selective binding to certain cell-types and lead to a strong increase in the preferential uptake of the chimeric proteins of the invention into selected cells. It is evident that these auxiliary domains can be multiple. For an example, additional sequences for a specific targeting of intracellular, non-chromosomal DNA such as mitochondria or parasite DNA (ie. virus, invertebrate, and bacteria infected cells) can be added. In another embodiment enhancer elements of the deliverers can be added.

A particulary preferred embodiment for a chimerical molecule of the invention is presented by SEQ ID No. 2, where the restriction enzyme PvuII with the two subunits connected as a single chain protein (SCPVU) represents the domain or region for the DNA binding affinity and for the DNA modification activity by the endonucleolytic activity and moreover the peptide SYGRKKR-RQRRRGGS of Tat contains the inter-cytoplasmatic delivery subunit.

One of the advantageous embodiments of the chimerical proteins of the invention is their stability also in cell culture-medium in the presence of serum. This stability can be finally increased by substitutions of amino acids in L configuration with D amino acids, or with non natural amino-acids, in the framework as it is technical possible in the art. These variants of the chimerical protein, as in the case of the point mutations exhibit the same enzymatic activity of the preferred restriction enzymes, or are missing the same nucleolytic activity in the molecules used for a control, and thus are contained within the invention presented.

Included also in the presented invention are the isolated polynucleotides that code for the chimerical molecules of the invention and as such are entirely or partially of recombinant nature and comprise SEQ ID No. 1 that codes for the chimerical single chain version of the PvuII enzyme for which the protein delivery region corresponds to SEQ ID No. 4 and is encoded by the nucleotide SEQ ID No.3. The preferred embodiment contains further a polyhistidine tag, that allows easy purification of the chimerical protein by nickel NTA affinity chromatography.

The invention contains further all the polynucleotide sequences that code for any of the possible realizations in which the chimerical molecule is a polypeptide and can be obtained with recombinant DNA technology with methods familiar to those skilled in the art as described for example in Sambrook and Maniatis, 1989, CSH ed. In agreement with these methods, and as also described for example for other restriction enzymes, for the peptides used as synthetic linkers or for peptides used for affinity purification, the techniques in the art is capable of producing realizations that show non essential differences to purification of the chimerical protein by nickel NTA affinity chromatography.

The invention contains further all the polynucleotide sequences that code for any of the possible realizations in which the chimerical molecule is a polypeptide and can be obtained with recombinant DNA technology with methods familiar to those skilled in the art as' described for example in Sambrook and Maniatis, 1989, CSH ed. In agreement with these methods, and as also described for example for other restriction enzymes, for the peptides used as synthetic linkers or for peptides used for affinity purification, the techniques in the art is capable of producing realizations that show non essential differences to the realisations of the presented invention and thus are included in latter.

Included also in the presented invention is a particular advantageous embodiment for the chimeric molecules of the invention and in particular for the nuclease activity impaired but DNA binding chimeric molecules, but also for RNA binding chimeric molecules. In this embodiment the DNA or RNA binding activity of the chimeric molecules can be used as a specific nucleic acid targeting molecule in a cell. This enables delivery of compounds as a chargo to the DNA or RNA of a cell. For this, specific compounds are covalently or not covalently linked to these chimeric molecules by recombinant techniques or chemical coupling. These compounds include for demonstration but not for exclusion: nanoparticles, inorganic or organic compounds and combinations there off, such as for demonstration but not for exclusion radioactive compounds, chemotherapeutics like doxorubicin bleomycin, vin-cristine, etoposide, cisplatin , and other radio mimetic and DSB inducing agents, antibodies and fragments thereof, colour compounds such as fluorescence molecules, natural and non natural nucleic acids, peptides or polypeptides containing or not containing enzymatic activities. Chemical coupling can be achieved with methods known in the art such as coupling of maleimide activated compounds to reduced cysteins in the chimeric molecules. For the case of chimeric molecules of the invention that do not contain a natural cystein, like for example PvuII, a cystein can be introduced by recombinant or synthetic techniques well known in the art. As a further example bromo-cyan coupling to free amino-groups can be used. A particular attractive coupling can be achieved by protein splicing and protein-ligation with selected compounds. Molecules can also be coupled to the various tags that are fused to the proteins for purification like for example a polyhistidine tag, a GST-tag or a protein A tag, myc tag, HA-tag, biotin, that allows to couple for an example but not exclusion antibodies, antibody-fragments or gutathione containing compounds. Molecules that are bound in this way to the proteins of the invention can or can not be cross-linked with chemical reagents and UV. For illustration but not exclusion, for these coupling procedures a particular attractive site in the chimeric molecules is presented by the N or the C terminal parts. In case of the single chain versions also the linker between the two subunits can be advantageously used.

In addition there are also the vectors included that contain the polynucleotide sequences described above, and in particular vectors for expression in prokaryotes which are generally more feasible for an expression of large quantities of recombinant proteins.

The use of the preferred chimerical molecules of the invention (where the DNA modifying enzyme is a class II restriction enzyme) allows its application in the main aspect of the invention that consists of a procedure to provoke, induce or generate DNA double strand breaks, at the palindrome sites that are cognate sites for a given enzyme, and thus advantageously for the sequences CAG/CTG (PvuII), GAT/ATC (EcoRV) or G/ANTC (HinfI) which are present randomly on the genome with a statistical frequency of approximately 6000 bp (EcoRV and PvuII) or 400-600 bp (HinfI).

The effect induced after treatment with the chimerical protein of the invention of cells in culture is detected by analysis of the cell cycle distribution by FACS analysis, or directly of the genome by Southern blotting, TUNEL assay, bromodeoxyurid-in labelling (BrdU), and by immunofluorescence using specific antibodies or green-fluorescence protein derivatives and their colour derivatives , all methods that can be applied routinely. Further, determination of clonogenic activities and the proliferation capacity of cells treated with the proteins of the presented invention represent an indicator for the functionality of proliferation controls, cell cycle controls and DNA repair.

The kinetics of the activity of the preferred embodiment of the proteins of the invention as measured for example by TUNEL assay is linear in the range between 0.001 nM and 100µM, more preferred between 0.1nM and 1µM, and even more preferred between 1 and 500nM. This linearity represents one of the advantages of the chimerical molecules of the presented invention, and in particular of SCPVUTAT, beside the monospecific activity and their feature to penetrate in all the cells. Moreover, events that are caused by the chimerical molecules do not have to be selected with specific selective agents and in turn simplifies noteworthy and advantageously the use of the latter.

The effects that are observed after the treatment with the chimerical proteins of the invention, and in particular with the preferred realization of the invention, reassume in a non limited mode as follows:
- increase of the percentage of the cells in specific phases of the cell cycle, such as G0-G1/S-S-G2-G2/M-M or in apoptosis or polyploidy. The quality of these changes is cell type specific and this specificity represents an ulterior diagnostic realisation of said procedure of the invention and allows to detect changes and/or genetic mutations or epigenetic/somatic changes in an unknown sample, compared to one or more controls, for an example, cell lines containing well described genetic defects or also normal cells. For an example the treatment of cells that contain a specific defect for the control of the cell cycle at the G1/S decision, like mutants in the gene for p21^{CIP/WAF1}, with said proteins of the invention exhibit an increase of the number of the cells in G2 in respect to the distribution seen upon treating of normal cells with said proteins of the invention. In the case of p21^{CIP/WAF1} cells do not stop in G1/S, but still exhibit a partially functional G2/M control point (checkpoint) and thus show a relative increase of the peak in G2/M phase, as can be seen in experiments where the distribution of the DNA content is analysed by FACS and compared to normal control cells. Similar results can be obtained from experiments of mutants in genes for gene-products that are involved in checkpoint controls for DNA repair such as ATM and Nbs.

As was shown before the importance of the method contains comparison of the distribution or comparison of behaviour of the cells relative to control cells (normal or control cells).
- changes in the steady state levels of key-proteins that are involved in the regulation of the cell cycle and repair. As a change in the steady state level of a protein variations in the amount of proteins are measured, for example this can result from variations in expression levels, or in changes in mRNA stability, or changes in protein modifications such as proteolysis, phosphorylation, ubiquitinylation or other posttranslational modifications.
- On the level of phosphorylation of proteins that are central in the ATM/ATR/DNA-PKcs control pathway and show changes after treatment with the said proteins of the invention. The proteins that exhibit changes in phosphorylation are better described in the experimental part that follows, but are herein listed in a non limited way: ATM (Ataxia-Teleangectasia Mutated protein), ATR (Ataxia-Teleangectasia Related protein), DNA-PKcs (DNA dependent Protein Kinase cathalytic subunit), and their direct or indirect substrates such as Chk1(Checkpoint Kinase1), Chk2 (Checkpoint Kinase 2), Brca-1 (Breast Cancer susceptibility 1), Brca2 (Breast Cancer susceptibility 2), Mre11 (Meiotic recombination protein 11), Rad 50 (Radiation 50 double strand break repair protein), Nbs (Nijemegen breakage syndrome), Rad51 (Radiation 51 double strand break repair protein), FANC-A, C, D2, E, F and G (Fanconi anemia complementation proteins), histones, helicases, BLM (Bloom's syndrome mutation), WRN (Werner's syndrome mutation), p53, etc.. In particular preferred are the molecular and biochemical markers: Nbs, Mre11, Rad51, p53, Chk2, Brca1.

In a particular specific embodiment is represented by the variations induced on p53 and direct or indirect transcriptional targets of p53 such as p21^{CIP/WAF1}, 14-3-3 sigma. The analysis of changes in the levels of these proteins or changes in activity and/or phosphorylation state of this proteins after treatment with the proteins of the invention represents an important aspect of the invention and is of particular interest for diagnostics. These measurements can be employed as it is well known in the art, for example by immunological methods (for example western-blotting) with specific antibodies for example for the phosphorylated form of the proteins like in the case for an increase in the phosphorylated form of p53 as detected with anti-p53-S15P. As it is demonstrated in more detail in the following experimental part, the treatment of different cell-lines with the proteins of the invention results in an increase of p53 protein and/or phosphorylation levels.
- changes of the cellular distributions of complexes where important proteins for cell-cycle controls or for controls that are important in the stress response in DNA repair. These variations can be observed from the cellular response in the nuclear foci where the Rad/Mre11/Nbs complex is localizing (Zhong Q. et al. 1999, Science 285:747-750), phosphorylation of Thr68 of Chk2, phosphorylation of ATM, phosphorylation of the Histone 2AX-Ser135 as analysed with immunological methods.
- Alternatively it is possible to follow the variations of the cytoplasmatic/nuclear distributions of the Cdc2/cyclinB1, Cdc25C, p21^{CIP/WAF1} and 14-3-3sigma complexes by comparing variations of mutant versus normal cells in response to treatment with the proteins of the invention. This can be done for example by fluorescent labelling specific for one of these components and analysis with the microscope as is described in the experimental example 12;
- apoptosis that can be measured with well known methods in the art, for example by FACS analysis, by cytocrome C release or caspase activities or by labelling with Annexin V specific antibodies. An increase in apoptosis for example can be detected by FACS analysis as an increase of the sub G1 DNA content in neuroblastomas after treatment of the cells with proteins of the invention.
- The effects that are induced from the treatment with proteins of the invention can be analysed also by mesasuring clonogenic activities or proliferative capacities with colour reactions well known in the art. Alternatively long-term effects of the proteins can be determined for example by the tumourogenic activity or invasiveness in animal model systems.

The selections of compositions capable to modulate genomic damage from the proteins of the presented invention can be analysed by sister chromatid exchange (SCE), analysis of ploidy, genetic amplifications, loss of heterozygousity and other assays well known in the art.

Finally, the invention applies for a method for measuring genetic predispositions for the development of tumours or genotoxic sensitivity (for example sensitive to radiation or intercalating agents), assayed in cells obtained from unknown samples (for example from biopsy) that includes essentially treatments with proteins of the invention, preferential in parallel with control cells, and successive measurement of the levels of expression or activation of either oncogenes such as for example myc, ras; or of tumour suppressor genes, such as for example ARF, p16, p53, Brca1 by specific assay reagents including immuno-enzymatic methods.

Whereas mutations from components in the cell cycle control system (checkpoint) leads to an increase in tumour-susceptibility, genetic defects in genes coding for the factors that are involved in DNA double strand break repair show lower penetrance, although if the combination of these defects with other risk factors, which can be for example an increase of the levels of reactive oxygen species (ROS), factors from chronically inflammation, can determine a mayor incidence in tumours.

In synthesis the invention concern certain different procedures all of which are characterised by the fact to use the polypeptides of the invention in cells in vivo, ex vivo or in vitro. In particular, the procedures according to the invention contain diagnostic procedures to evaluate a genetic damage co-involved in cell cycle controls and in DNA repair and also procedures for a selection of compounds with a biological activity that is capable to modulate these control activities. Some diverse aspects of the invention are based on the biological activity of the chimerical polypeptides of the invention that, due to the induction of a DNA double strand break with above described specificity activate control pathways for the cell cycle and for DNA repair (checkpoint).

As is observed and described in more detail in the example section, and as a consequence of the induction of monospecific DNA double strand breaks, the polypeptides of the invention exhibit therapeutical activity, and in particular antiproliferative and antitumourogenic. This activity therefore represents further subject of the invention. Thus the invention includes pharmaceutical compositions containing as an active substance the polypeptides or polynucleotides that encode these polypeptides, as described in the invention. Moreover, the invention includes the use of the chimerical polypeptides of the invention and/or the polynucleotides that encode these polypeptides, for the preparation of pharmaceuticals for the prevention, the therapy or the diagnosis of neoplastic disease or predisposition to this disease.

A further embodiment of the invention includes the diagnostic use of the polypeptides or the polynucleotides from the invention in the diagnosis of tumour pathology or for diagnosis of a predisposition for these diseases as described in detail in the example section.

As a preferred embodiment treatment of the cells with the proteins of the present invention, for example for its use in diagnosis and employed as described above is applied for the test-samples and in parallel for cell-lines that do not contain the putative mutation (control). In an even more preferred embodiment of the assay additional control treatments are included in the experiments with the chimerical proteins of the invention by the use of test cell-lines that contain for example mutations in selected steps of the DNA repair pathways which allows by comparison, a fine mapping of respective genetic changes.

After a longer time period, such as after 24 hours after administering of the proteins of the invention it is possible to detect a differential effect depending if cells are able to repair the genetic damage (DNA double strand break, DSB) that was induced by the proteins of the invention and if in such a case the distribution of the cell populations in the various phases of the cell-cycle returns to normal or not. In cells with mutations of the gene encoding for ATM or for example in other genes that code for products that are involved in the NHEJ (Non Homologous End Joining) DNA repair pathway apoptosis can not be observed immediately but constitutes an indirect effect with low incidence. In contrary, in neuroblastoma cells apoptosis is induced immediately and in a direct fashion. Overall the invention includes in a further embodiment the use of the chimerical proteins and polynucleotides encoding this proteins to induce apoptosis in cells of neuronal origin, and preferred for neuroblastoma and thus for the preparation of a pharmaceutical for the treatment of tumours of neuronal origin.

The induction of apoptosis in neuroblastomas correlates with 1p36 deletion in combination with double minute (DM) myc-N oncogene amplification. This hypersensitivity does not correlate with the presence of the 17q15 translocation. DM myc-N amplifications are episomal myc-N clusters, known as the most aggressive forms of myc-N amplified NB and have extremely low prognosis. Great part of the aggressive malignant NB exhibit this type of oncogenic amplification. In another particular embodiment other tumours that contain DM amplifications of oncogenes such as for illustration but not exclusion, treatment of DM containing prostate or breast tumours is also included. In general chimeric molecules that are coupled to said compounds can be used for the preparation of a pharmaceutical for the treatment of selected tumours.

The cellular response to a treatment with the proteins of the invention is different from cells that either have a mutation in DNA repair pathways or exhibit changes in components that are responsible for the control of these pathways for cell cycle and/or repair (checkpoint). Therefore the presented invention contains a series of procedures, all of them substantially characterized by the fact to use the induction of DSB in the genome of a test-cell by applying the preferred embodiment of the chimerical molecules according to the invention and to analyse the responses with assays as described above, preferential by comparison of the results from the test sample with standard cells of most possible isogenic nature.

The effects of the proteins of the invention on the cell cycle and mechanisms for DNA repair are synergized in the presence of inducers of free radicals (ROS), this can be for example H₂O₂. This synergistic effect is also observed at very low concentrations of the chimerical protein, preferred below 10nm of the SCPVUTAT protein and below 10µM H₂O₂. Therefore the invention contains a procedure for an induction of DSB in the genome that is characterized by the fact that the proteins of the invention are used in combination with reactive oxygen producers (ROS), for example H₂O₂. This process can also be used for a selection of antagonistic or synergistic compounds.

Induction of DSB in a cell in vivo activates a cellular and molecular response similar to those induced by ionising irradiation frequently used for anti tumour therapy; but compared to this shows reduced collateral effects. Thus a further embodiment of the invention contains the use of the chimerical polypeptides from the invention for the preparation of pharmaceuticals with anti-tumour activity or for the therapy, the diagnosis or the prevention of genetic disease which in turn define the predisposition of an individual to develop diseases caused by a deregulation of the proliferation activity of a cell and in particular neoplastic disease which includes essential gene products for the mechanisms for the control of DNA repair.

Based on this aspect the invention comprises a therapeutic method based essentially on the administration in vivo or ex vivo of the chimerical polypeptides of the invention as defined above, where an antiproliferativ effect, preferred anti tumourigenic is needed.

A further embodiment of the invention contains a procedure for the in vitro diagnosis of a genetic or somatic defect in DNA repair or in defects of the regulation of the cell cycle from isolated cells obtained from a biological sample and consists essentially of the following:
a) Growth of isolated cells in culture of which one intends to measure efficiency or damage in the DNA repair pathways or cell cycle pathway.
b) Incubation of these cells with the preferred molecule of the invention, where the modifying enzyme is class II endonuclease, and more preferred the single chain version of the PvuII enzyme (SCPVU). Advantageously in parallel a treatment of the cells is also done with a control polypeptide that exhibits specific DNA binding activity but is impaired for nuclease activity like for example SC34. Optional, further a parallel treatment of feasible cell-lines that are mutated and/or defective in DNA repair, the controls of DNA repair and/or of the cell cycle, whereas these lines are preferred of most isogenic nature;
c) Characterisation and measurement of the cellular responses;
d) Optional comparison to a control cell-line.

For isogenic two or more cell-lines are meant that exhibit an as possible most similar genetic background like for example the cell lines: MRC-5 and AT-5; CHO-K1 and KU70 o MO59K and M059J as described and used in the experimental examples below. The standard-control cell-line can also be a normal cell-line with no changes in the control pathways of the cell cycle and of DNA repair, and should be most possible isogenic to the test-sample, or a cell-line with a well characterized genetic change.

In a preferred embodiment of the procedure of the invention the test cells and the control cells are grown together and under identical conditions. Thus they are maintained and compared in a direct mode, differences in cultural markers, of those for example : clonogenic capacity, % of cells in apoptosis or quiescence, as measured for example by the determination of the proliferative capacity with live cellular colours, or by measurements of biochemical markers, of these described above. An example for this type of assay is described in Torrance C. J. et al., 2001, Nature Biotechnology, 19, 940-945.

In a preferred embodiment for the characterisation of the response described in c) a measurement of the clonogenic activity is used, as is well known in the art, such as these for example by growth of cells in culture or by measurement of the clonogenic activity with growth in soft-agar, or by FACS analysis after DNA staining.

A particular useful embodiment described in the invention and of particular usefulness for diagnosis and/or prognosis to evaluate: genetic predisposition for development of neoplastic disease, sensitivity in tumour therapy, in particular tolerance towards radio- or chemotherapeutic antitumour treatments of a patient, or also sensitivity of tumour tissue in comparison with healthy tissue for prognostic reasons.

The invention contains beside also kits to carry out the procedures of the invention, thus preferred diagnostic kits or kits for research.
Some preferred embodiments of the kit contain :
1) a tube containing the chimerical polypeptide, preferred the chimerical endonuclease chosen between EcoRV, PvuII or HinfI as a single chain version in fusion with the Tat delivery peptide, or an expression vector that contains the polynucleiotides that code for the chimerical proteins, in combination with a tube that contains the control-polypeptide a chimerical protein that exhibits specific DNA binding protein but is impaired in the endonuclease activity, or an expression vector that contains the polynucleiotides that code for this chimerical protein. In a preferred embodiment the kit consists of tube that contains the chimerical protein SCPVUTAT and of another tube that advantageously contains the chimerical protein SC34 in lyophilised form or in an aqueous solution;
2) the kit optionally contains a tube with an antibody against the entire or parts of the chimerical polypeptide, preferred an anti-SCPVUTAT antibody;
3) the kit optionally contains a tube or a flask containing control cells as defined above, for example cells that are hypersensitive to DSB or cells that contain well characterized mutations in genes coding for proteins that are involved in the DNA repair and cell cycle pathways and their controls (checkpoint). Such cells might be contained as frozen stocks or in flasks adapted for transportation;
4) the kit optionally contains chemotherapeutical reagents like intercalating agents, radiomimetics, or other types of chemicals including pharmaceuticals for determination and comparison of the qualitative and quantitative effects.

As a further important embodiment that is based on the activities of the preferred chimerical polypeptides the invention contains a procedure to select for compositions that modulate, are synergistic, antagonistic or do not change DNA repair activity and/or controls of the genome and for genomic stability in a cellular system by using high-throughput screens based on cells, including essentially as follows:
a) incubation of test cells with the chimerical proteins of the invention preferential in the presence of an appropriate control polypeptide (in the specific case consisting of a chimerical molecule that contains specific DNA binding activity but exhibits impaired endonuclease activity such as the chimerical polypeptide SC34), optional also in the presence of synergistic substances such as for an example H₂O₂ or other radical producers and/or other modulating or antagonistic substances;
b) optionally in parallel the incubation is also done with other, control cells that are as much as possible isogenic with the test cell, for example the same cells containing a reporter gene. Various reporter genes are well known to those in the art , among them for example the EGFP proteins and their mutants as described for example in Torrance et al., 2001, Nature Biotetechnology, 19, 940-945; or other types of reporters that allow an efficient automatic readout of the assay;
c) addition of compositions which are supposed to be tested for a potential activity or no activity, among them for example single molecules or chemical libraries or collections of chemicals, peptides or others or collections of biological samples;
d) the read out of the cellular response is preferential done in an automatic mode, and preferred with high-throughput screening (HTS) facilities. Examples for this are systems that are based on the measurement of variations of a morphological phenotype, like a fluorescent signal induced as a response to control substances and positive test-substances that show biological activity in a cell. The cellular response can consists of any cellular signal or signal from included biochemical markers, or also the detection of cellular parameters such as for example calcium flux, radical flow, change in cytocrome C, combined with a system that allows automatic detection.

During this procedure of the invention the order of the steps b) and c) might be inverted.

This assay allows for selection of compositions that contain important biological activities for the control of the cell cycle, DNA repair pathways, induction of apoptosis, induction of senescence, or by interrupting one pathway that in turn causes activation of another pathway.

Further selection cycles can be applied after advantageously modification of the isolated compounds from the first rounds of a screen, to finally obtain compositions that are pharmaceutical more adapted or also to select for additional advantageously features such as for example bio-compatibility or product stability.

Besides further aspects derived from the above described, the invention contains a method for inducing cell-cycle blocks or alternatively to induce apoptosis preferred in cells of neuronal origin or alternatively to induce DNA repair in isolated cells, based on a use of the chimerical molecules of the invention, preferential represented by class II restriction endonucleases, and even more preferred as a single chain version, or even more preferred by the enzyme SCPVUTAT, as well as polynucleotides that code for these molecules, and where this effect is synergized in the presence of compositions such as for example free radical producers. These methods are essentially based on the principal to induce DNA double strand breaks by the chimerical polypeptides of the invention.

A further embodiment of the invention contains a procedure for the selection of new penetration sequences and the selection of auxiliary sequences. In this embodiment the penetration or auxiliary sequences do not consist of a singular compound but compound library molecules are used to select for putative penetration or auxiliary domains. These procedures are well applicable with high throughput screens that enable analysis of many different samples. The principles described in the invention for diagnosis are also the basis for screening procedures that allow the discovery of new penetration sequences and auxiliary sequences basically for any celltype or organism. Automatic and robotic devices allow performance of the steps below with high-throughput capacities. This can be done with pippetting and readout units known in the art. For illustriation but not for exclusion for a screen for specific auxiliary or penetration sequences consist essentially of the following steps:
1. For specific auxiliary sequences the penetration sequence is constant and libraries of the chimeric proteins of the invention are constructed with methods in the art. For an example, to the N terminus of the chimeric proteins of the invention various light chain and functional CDRL fragments are fused and then mixed with the same proteins of the invention that are fused to variable heavy chain immuno-globulin proteins including functional CDR1-3H regions. This is obtained by recombinant techniques in a two plasmid expression system preferentially in E. coli. In general libraries of immunoglobulin or fragments of immunoglobulins, or receptor binding proteins or random libraries produced from synthetic or natural origins can be linked to the proteins of the invention by recombinant techniques or other coupling methods as also described above. For example for illustration, antibody-fragment libraries can be linked to the proteins of the invention by binding to protein A contained in the molecules of the invention.
2. For the case of a penetration sequence screen libraries can be obtained by techniques well known in the art. For example these libraries are obtained from natural origins or synthetic origins including compounds of any class. For a particular advantageous embodiment bacterial penetration sequences are screened from random DNA fragments from the same or from different species.
3. The library protein expressing bacterias are grown and induced for protein production. These proteins can be isolated from the supernatants from secreting systems but also from cell lysis followed by high throughput affinity preparation of the chimeric proteins using for example various affinity tags like for illustration a polyhistidine tag, a GST-tag, a protein A tag, a myc tag a HA-tag, or biotin.
4. These proteins are used to analyse for function or differential function of a given molecule from the library. If sequences are functional the chimeric proteins are functional. This is the case when they are able to cross the cellular and eventually nuclear membranes and bind to the DNA and occasionally introduce DSBs. Or for the auxiliary screen, when function can be detected in selected cells. Detection can be done like described above.
5. Test cells or tissue can be of any origin including prokaryotic cells where the function of the chimerical proteins of the invention can be detected in a mode as for example described above.
6. In a particular useful embodiment test cells are stably transfected with EGFP (enhanced green fluorescence protein) for an easy read out;
7. In another particular useful embodiment control cells are differentially stably transfected for example with EYFP (enhanced yellow fluorescence protein);
8. Test and control cells are either grown separately or mixed in multi-well devices or on specific membranes. Cells can be very similar with only single changes (ie. Used to find auxiliary sequences such as tumour and no-tumour cell from the same origin) or very diverse (ie. Test cell is were delivery should occur but for control cells no delivery must take place; this includes also cells from different species such as test cell is a bacteria cell and control cells are of human origin). The choice of the cells allows any technically possible combination.
9. Proteins are added to the supernatants of the cells and incubated;
10.Read out will be for function of the chimeric molecule as described in the examples above. In a particular advantageous example detection is for green and yellow cells prepared as above. Proteins that produce significant more yellow cells than green cells are further isolated as single clones, by back-screening and analysis with recombinant and mass-spec procedures well known in the art. In a preferred application where the chimeric molecules introduce DNA damage after entering into the cells, this assay can be made even more sensitive. Test-cells (ie. Tumour cells) and the control-cells (i.e. compareable non tumorigenic) are rendered hypersensitive to DSBs introduction by gene-disruption or gene-silencing or introducing dominant negative or dominant gain of function mutants. This is obtained by methods well known in the art such as by cotransfection with siRNA, by vectors expressing siRNA's, targeting vectors for gene disruptions or mutagenesis, vectors for expression of mutants or overproduction of proteins that inhibit important functions in DNA repair and show hypersensitivity to DSB, chemical inhibitors or activators. This allows faster and more differentiated read out at low concentrations. For example siRNA inhibition of Ku70, Ku80, DNAPKcs, ATM, XRCC4 and Ligase IV, Bc12. But also inhibition of ATM by chemical compounds such as caffeine. P53 or other apoptosis regulating factors can be introduced.
11. For further final confirmation of the sequences from the screen again, preferentially automatic steps are used:
   - Clones are confirmed with pure proteins
   - Concentrations are titrated;
   - Testing of selected sequences in nuclease dead versions;
   - Test of other cells;
   - Test in mice for toxicity.

The present invetion is further described by the following examples and the drawing figures, yet without being restricted thereto.

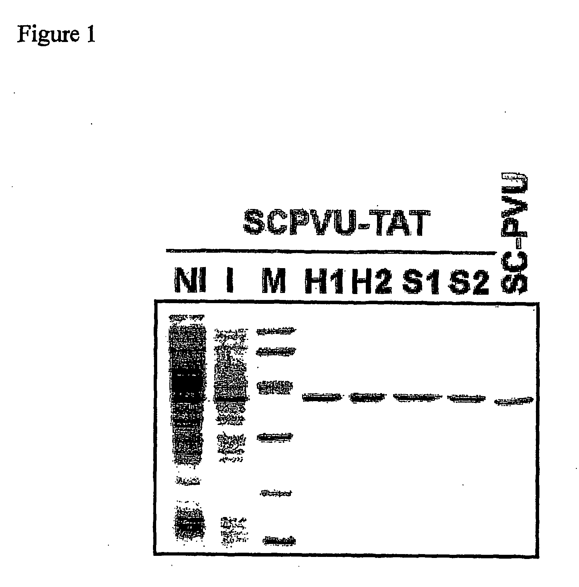

### Figure 1. Purification of SCPVUTAT from E.coli.

### Expression and purification of SCPVUTAT.

E. coli cells that contain the expression plasmid for SCPVUTAT were induced for the expression of the protein and the purification was done as described in the experimental part. NI and I represent the total cellular extracts from E.coli not induced or induced respectively. H1 and H2 are peak fractions obtained from the purification on the Hi Trap chelating Ni⁺⁺ agarose column; S1 and S2 are the peak fractions obtained from the purification on a SP-Sepharose column; M represents molecular mass markers (from the top to bottom in kDa: 94, 67, 43, 33, 20, 14); SCPVU represents the protein that does not contain the TAT sequence and was purified in a similar way.

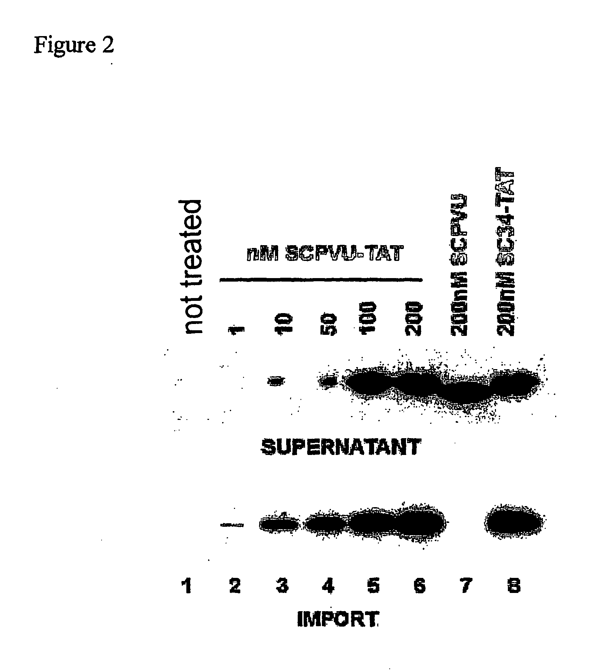

### Figure 2. Immunological analysis of the protein-extracts from U2OS cells after protein transduction.

The analysed proteins were: increasing concentrations of SCPVUTAT (1, 19, 50, 100, 200nM; lanes 2-6), SCPVU (200nM, does not contain the TAT sequence, lane 7), SC34 (200nM, SCPVUTAT derivative that exhibits no enzymatic activity, lane 8), control (no addition of proteins to the cells, lane1). Ten minutes after the addition of the proteins to the cell culture supernatants, extracts were prepared after extensive washing with PBS, separated on SDS-PAGE and analysed by immunobloting with monospecific antibodies to PvuII (lower panel, IMPORT). For a comparison aliquots of the supernatants of the cell culture taken before extract preparation were included in the analysis (upper panel, SUPERNATANT).

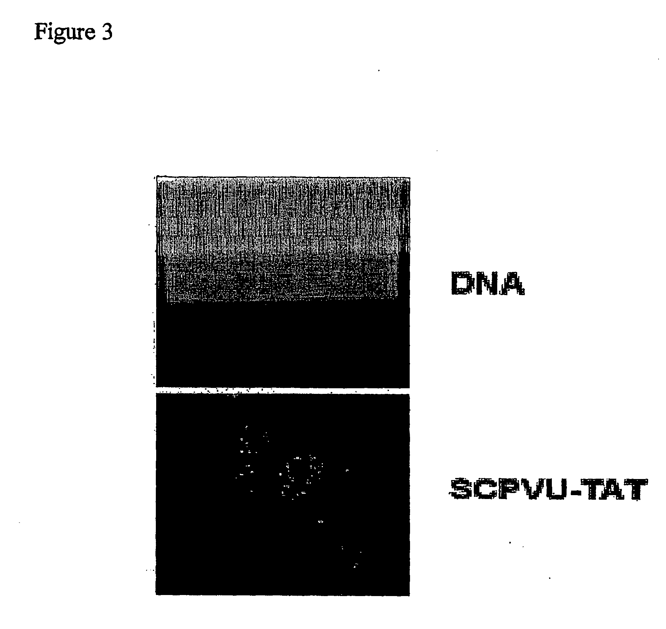

### Figure 3. Immunofluorescent analysis by confocal microscopy.

After 30 minutes of proteintransduction with SCPVUTAT cells were washed, fixed with 3% PFA, marked with PvuII monospesific antibodies, and stained with FITC conjugated secondary antibodies for confocal Immunofluorescent analysis (green,lower pannel). In addition DNA costaining was obtained with propidium J⁻ after RNAse A digest (red, upper panel).

### Figure 4. TUNEL analysis for detection of DSB on a single cell level.

U20S cells were treated with SCPVUTAT and DSBs were analysed with the confocal immunofluorescence microscopy after labelling with TdT in the presence of dUTP-FITC (TUNEL). Treatment was with 100nM SCPVUTAT for the timeperiods indicated.

### Figure 5. Nuclease dependent cell cyle delays induced by SCPVUTAT.

The distribution of the various phases of the cell cycle of the protein transduced cells was analysed by FACS analysis. Distribution of the DNA content of the cell populations was detected. 2n represents a diploid DNA equivalent (non replicated; G1 phase), 4n represents the tetraploid DNA equivalent (replicated; G2/M phase). For the FACS analysis the cells were separated employing the DDM mode that allows to estimate DNA content of a single cell. U20S cells were grown for 24 hours (upper row) or 48 hours (lower row) in the presence of increasing amounts of SCPVUTAT (10nM, 50nM, 100nM) as indicated and with only one addition of proteins; non treated cells (control); cells treated with a nuclease impaired derivative of SCPVUTAT (SC34, 100nM). All the histograms shown represent a measure of DNA content relative to the cell numbers as indicated only on the upper right panel.

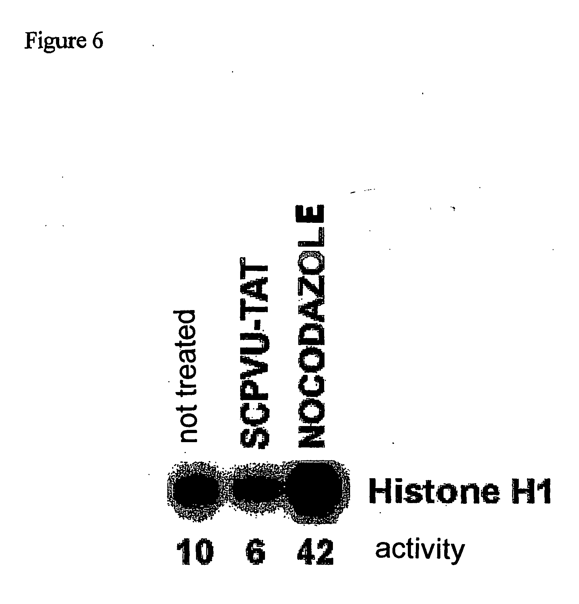

### Figure 6. Kinase activities induced by SCPVUTAT.

Determination of the cyclin B1 kinase activity using histone H1 as a substrate and cyclin B1 specific immunoprecipitates from protein extracts obtained from growing U20S cells, not treated or treated with SCPVUTAT (100nM), or treated with nocodazole (0.2 µg/ml) for 30 hours. Cyclin B1 specific immunoprecipitates were incubated with histone H1 in the presence of Y³²P-ATP and the reaction mixtures were separated on SDS-PAGE and analysed by autoradiography. The numbers on the bottom indicate the relative activities as determined by phospho-imager analysis.

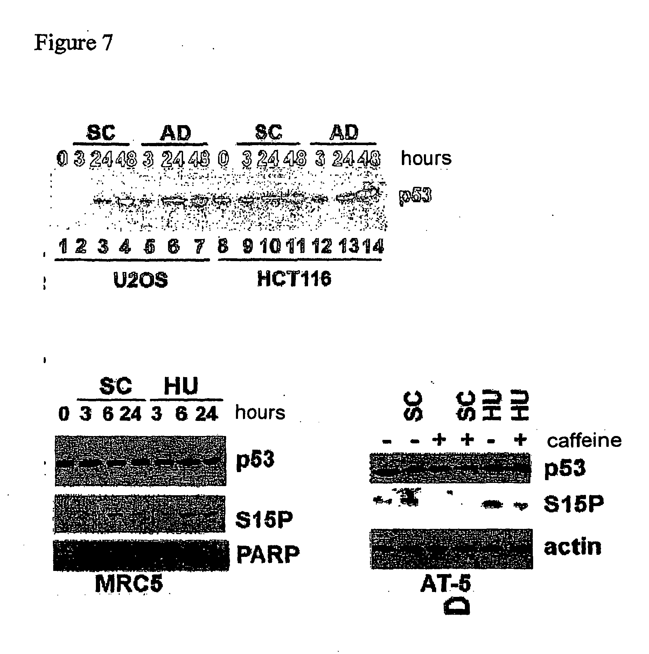

### Figure 7. Biochemical markers for the cell cycle arrest after SCPVUTAT treatment.

A) Induction of p53 by 3'-OH and 5'-phospate containing blunt end DSBs. U20S cells (lanes 1-7) and HCT116 (lanes 8-14) were treated with SCPVUTAT (100nM, SC), or with adriamycin (doxorubicin, 0.02 pg/ml, AD) for the timeperiods as indicated; non tretated cells were used as a control (lane 0). Extracts were prepared, separated on SDS-PAGE and analysed by immonobloting with specific antibodies to p53 (upper panel, p53) or to PARP (lower panel, PARP).
B) Repair response of the Ataxia telangectasia mutated (ATM) protein to SCPVUTAT. SCPVUTAT dependent Ser¹⁵ phosphorylation of p53. The ATM positive cell-line MRC5 was treated with SCPVUTAT (100nM, SC) or with hydroxyurea (1mM, HU) for the time periods as indicated; as a control non treated cells were used (0). Extracts were prepared, separated on SDS-PAGE and analysed by immonobloting with specific antibodies to p53 Ser¹⁵ (upper panels, Ser15, p53), or to actin (lower panel, actin).
C) Repair response of the Ataxia telangectasia mutated (ATM) protein to SCPVUTAT.

Caffein sensitivity of SCPVUTAT dependent Ser¹⁵ phosphorylation of p53. AT-5 cells, negative for ATM were treated with SCPVUTAT (100nM, SC) or with hydroxyurea (1mM, HU) in the presence of caffeine (2mM) for 4 hours; as a controls cells without treatment and cells only with caffeine treatment were used. Extracts were prepared, separated on SDS-PAGE and analysed by immonobloting with specific antibodies either to p53, p53 Ser¹⁵ (upper panels, Ser15, p53) or to actin (lower panel, actin).

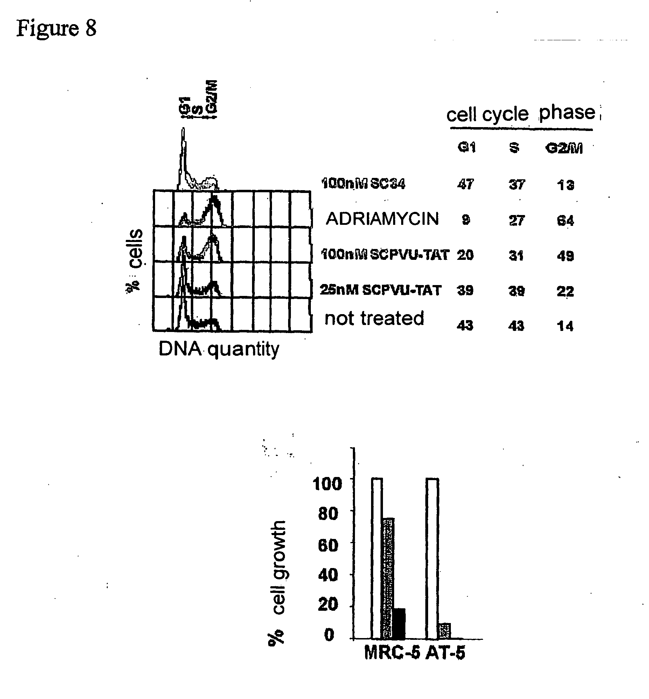

**Figure 8**. Induction of cell cycle block and clonogenic activity by SCPVUTAT of cells mutated for ATM (AT-5) in comparison to non mutated cells (MRC-5).
A) Distribution of DNA content distribution during the cell cycle detected by FACS analysis of AT-5 cells after 36 hours of SCPVUTAT treatment (25nM, 100nM), or with adriamycin (0.02µg/ml; ADRIAMYCIN), or with SC34 (100nM), and as control from non treated cells. The histograms represent DNA content relative to the cell-numbers; the panel on the right indicates the percentage of the corresponding distributions of the various cell cycle phases as calculated with the MODFIT^{™} program-package.
B) Colony forming assay with cell lines mutated for ATM and induced for DNA damage by SCPVUTAT. Various dilutions of AT-5 or MRC-5 (serves as an ATM positive control) were treated for 60 minutes with SCPVUTAT (25nM, grey bars; 100nM, black bars) or not treated (white bars); the cells were washed and then grown for 7-10 days, stained with GIEMSA and colonies were counted. A summary of the results is shown by histograms and represents the medium of three independent experiments with standard deviations in the range of 10%-15% as shown. Non treated cells were set to 100%, the black bar for AT-5 cells is closed to 0% and thus was not indicated.

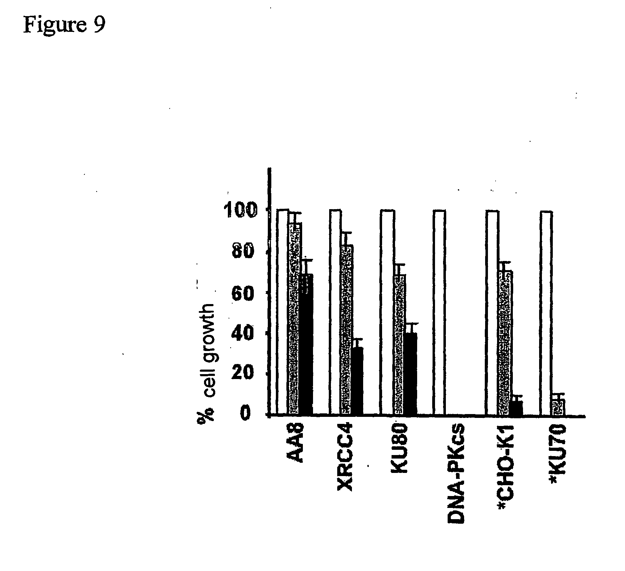

### Figure 9. Comparison of the clonogenic activities of cells with selected mutations in single proteins involved in the NHEJ pathway.

Clonogenic assay of CHO cells that were mutated for proteins involved in the NHEJ repair-pathway treated with SCPVUTAT. The cell lines used are AA8 (parental line), V3 (DNA-PC_{cs}^{(-/-)}), xrss5 (KU80^{(-/-)}) and XR-1 (XRCC4 ^{(-/-)}), HIS P1.13-11 (KU70 ^{(-/-)}) and the corresponding parental cell line CHO-K1. Various dilutions of the CHO cells containing the NHEJ mutations as indicated were treated for 24 hours with SCPVUTAT (25nM, grey bars; 100nM, black bars) or not treated (white bars); the cells were washed and then grown for 7-10 days, stained with GIEMSA and colonies were counted. A summary of the results is shown as histogram and represents the medium of three independent experiments with standard deviations in the range of 10%-15% as shown. Non treated cells were set to 100%, the black bar for the cells V3 (DNA-PC_{cs}^{(-/-)}) is below 1% and thus was not indicated.

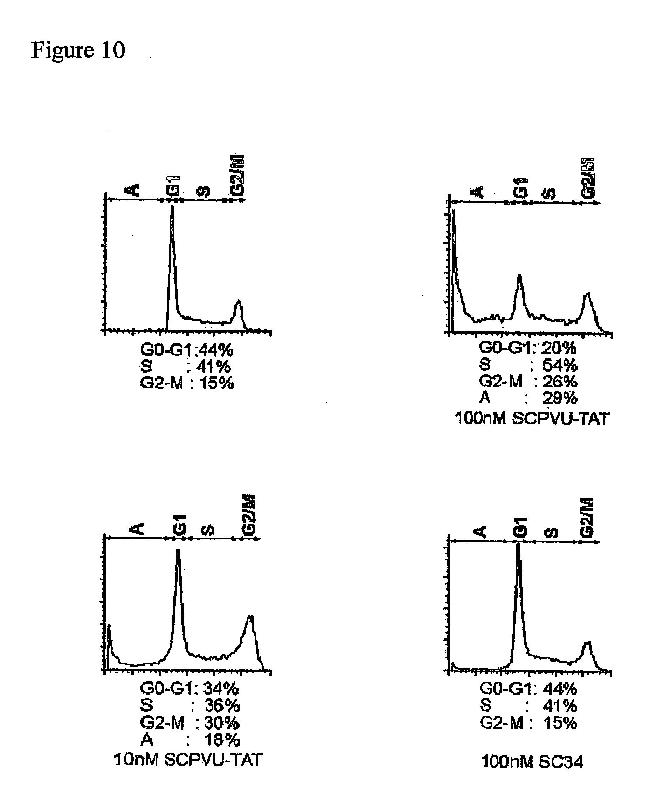

### Figure 10. Differential induction of apoptosis in neuroblastoma cells after SCPVUTAT treatment.

FACS analysis of the DNA content stained with propidium J⁻ of the neuroblastoma cells GI-LIN after SCPVUTAT treatment (10nM, panel C; 100nM panel B). The treatment of the samples was done for 30 hours. As a control samples treated with SC34 were used (100nM, panel D) or not treated (panel A). At the bottom of each panel the representative percentage of the cell cycle phase distributions obtained are indicated (G1/S/G2-M). (A) indicates apoptotic cells.

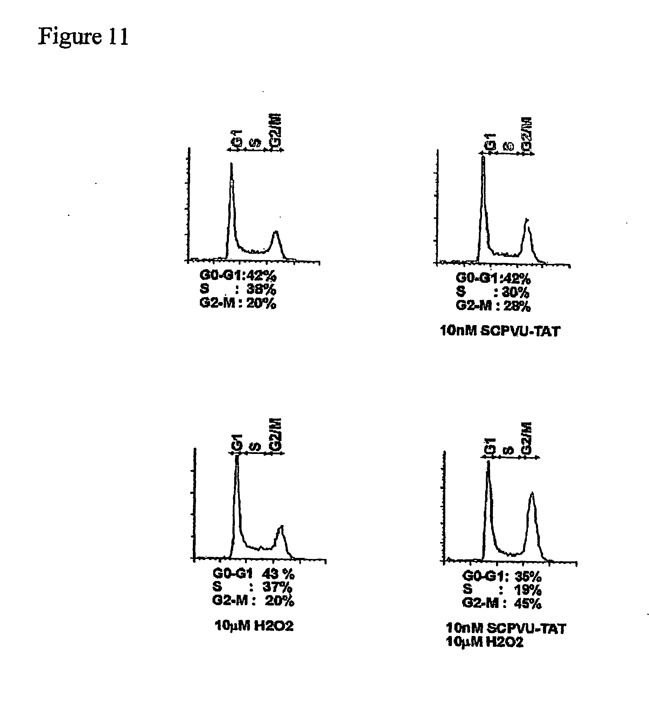

### Figure 11. Synergistic effects of low concentrations of SCPVUTAT and sub-lethal concentrations of H₂O₂.

U20S cells were incubated with SCPVUTAT (10nM, panel B), or with H₂O₂ (10µM, panelC), or both (SCPVUTAT, 10nM; H₂O₂, 10µm; panel D), or with no agent as a control (panel A). Further treatment cells were analysed in FACS for DNA content; the percentage of the various cell cycle phases are indicated at the bottoms of the individual histograms.

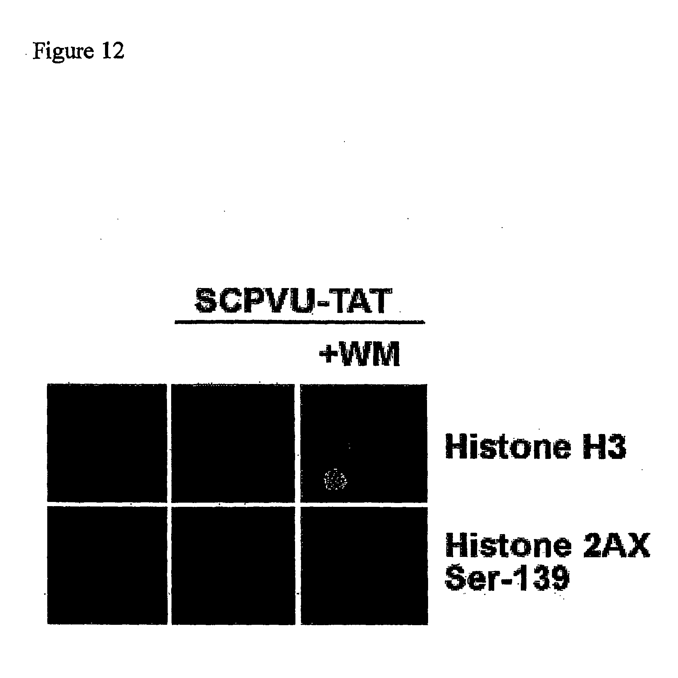

### Figure 12. Microscopic analysis of histone 2AXSer-139 posphorylation and the inhibition of this effect by the PI3/ATM kinase inhibitor Wortmanin.

U20S cells were treated with SCPVUTAT (100nM, central column) for 60 minutes, in addition treatment was done with Wortmanin (50µM, WM, right column), or with no agent as a control (left column). After treatment cells were stained with specific antibodies to H2AX-Ser-139 phosphorylated (red) or for Histone 2B (served as a control, green) and analysed on a single cell level by fluorescence microscopy.

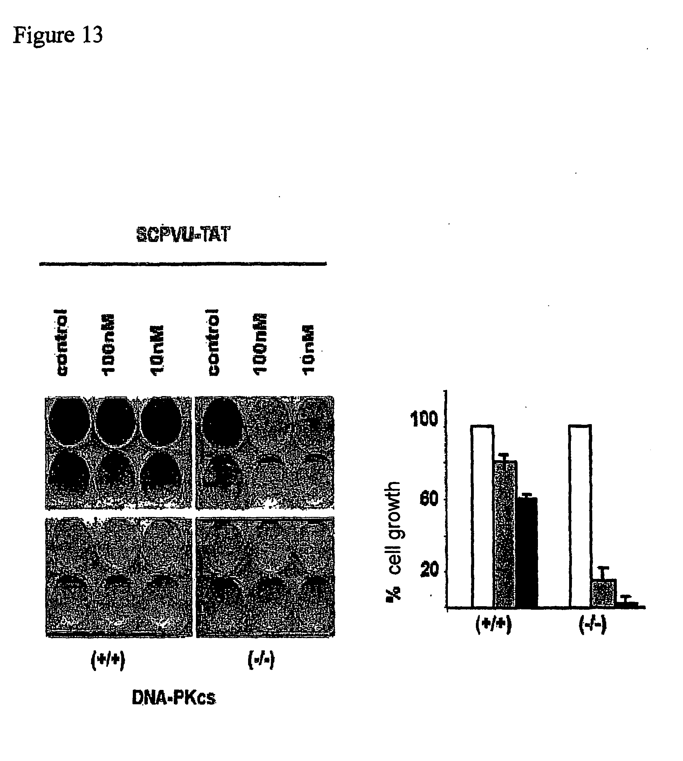

### Figure 13. Determination of hypersensitivity of cells mutated for the catalytic subunit of DNA dependent protein kinase (DNA-PK_{cs}) by automatic analysis.

A) The proliverative capacity of mutated cells and of comparable normal cells was evaluated with a Versadoc 4 (BioRad) imaging device using coloured cells. Statistical analysis of the results was done and is represented as a histogram.
   Colony forming assay with the human glioblastoma cell lines MO59J (DNA-PK_{cs} ^{(-/-)}) compared to M059K cells (DNA-PK_{cs} ^{(+/+)}) after SCPVUTAT treatment. An example of the colony forming assay is shown. The assay was done employing various concentrations of cells in the order of three magnitudes (from the upper to the lower rows, 1x10⁴, 2x10³, 4x10², 8x10). On the right B), the histograms of the results from the colony forming assay of the cell-lines MO59J compared to M059K is shown. The assays were done as in Figure 9 but analysed with the aid of the Versadoc 4 (BioRad) system. White bars: samples not treated; grey bars: 25nM SCPVUTAT ; black bars: 100nM SCPVUTAT.

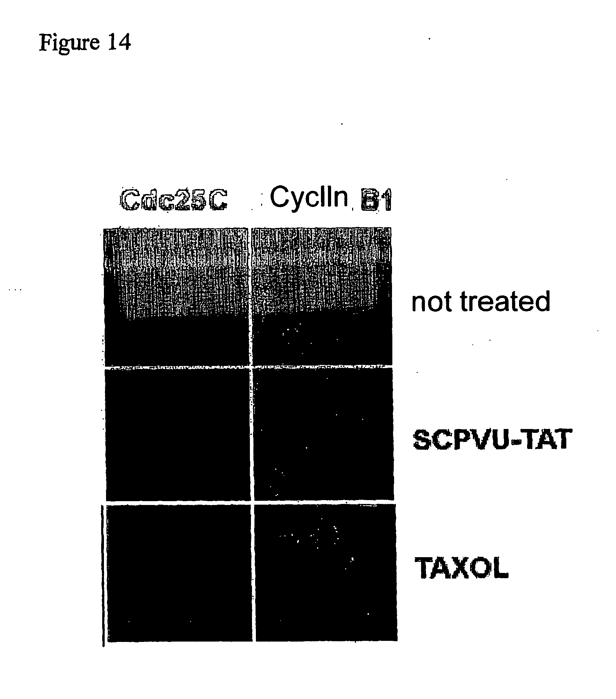

### Figure 14. Intracellular distribution of cell cycle control proteins after a treatment with SCPVUTAT.

Microscopic analysis of the HCT116 (p53(+/+)) cells not treated, or treated with SCPVUTAT (100nM) for 30 hours, or with taxol (0.2µg/ml). Immunofluorescence analysis was done with primary antibodies specific for cyclin B1 or specific for Cdc25C and coloured with secondary conjugates of. FITC (green, cyclinBl) or TRITC (red, Cdc25C).

### EXAMPLES

### Example 1

### Vector construction for the expression of the recombinant proteins

For the experiments described in the following examples the following recombinant proteins were produced using methods well known to the expert in the field: SCPVU, a single chain variant of the homodimeric endonuclease enzyme PvuII, already described in Simoncsits et al., 2001. To the C-terminus of this construct was added the sequence coding for GSYGRKKRRQRRRGGSHHHHHH containing part of the HIV Tat protein and is followed by a six Histidine tag. The recombinant fusion protein obtained in this way is called SCPVUTAT. This results in a polypeptide that is composed of the amino acids 1-157 of the enzyme PvuII followed by a lincer with the sequence -GSGG which connects the first subunit to the second subunit of the enzyme PvuII (aa 2-157), and is followed by the sequence GSYGRKKRRQRRRGGS-HHHHHH (tat-peptide + 6 histidine-tag). Further, the variant SC34 of the endonuclease PvuII (Simoncsits et al., 2001) is produced as a single chain polypeptide. This derivative exhibits the specific DNA binding activity of PvuII, but is impaired in endonuclease activity due to a mutation (Asp34/Gly34) at position 34 in both subunits of the PvuII enzyme.

### Example 2

### Expression of the recombinant proteins and their purification.

The expression of SCPVUTAT was done in the E.coli strain XL1 MRF' (Simoncsits et al., 2001) and the protein was first purified on a HiTrap Chelating affinity column (5ml, Amersham Pharmacia Biotech) and then further purified on a SP Sepharose (5ml HiTrap SP HP, Amersham Pharmacia Biotech). On the SP-Sepharose proteins were eluted between 0.63 M and 0.67 M NaCl. Yield was approximately 10 mg of purified protein from 1.5 1 of medium. The native PvuII protein (not as a single chain version) fused to the TAT sequence was prepared in a similar way. In this case elution from SP-Sepharose was between 0.81 M and 0.85 M NaCl. All of the expressed proteins were purified to homogeneity as judged by gel-electrophoresis on an 15% SDS-PAGE and mass spectrometry with an API 150 EX (Perkin Elmer) confirmed the theoretical mass. The enzymatic activities of the endonuclease derivatives obtained was compareable to the native enzymes as was assayed with λ DNA as a substrate.

### Example 3

### Production of antibodies and immunological analysis.

Antibodies against recombinant proteins were raised and used by standard methods as described for example: "Using Antibodies: A Laboratory Manual", Ed Harlow, and David Lane; CSH press New York, 1999, ISBN 0-87969-544-7, "Cells: A Laboratory Manual", David L Spector, RobertD. Goldman; Leslie A. Leinwand; CSH press New York, 1998, ISBN 0-87969-521-8. The antibodies were obtained from immunisation of New Zealand white rabbits with proper antigen and sera were purified against corresponding antigens that were immobilized to BrCN-Sepharose (Amersham Pharmacia Biotech). Cellular extracts for immunoblotting were obtaind by lysing of the cells in 20mM Tris HCl pH 8.0, 5mM EDTA, 150 mM NaCl, containing the protease inhibitors for example 20 µM TPCK, 20 µM TLCK, phosphatase inhibitors 60 mM 4-nitrophenyl phosphate, or by direct lysis in SDS sample loading buffer. Immunofluorescence analysis was done by methods known in the art for example after fixation of the cells in 3% paraformaldehyde or fixation in a 1:1 mixture of acetone and methanol. Analysis was done after antibody-staining using a Zeiss Axiovert 100M microscope attached to a LSM510 confocal unit.

### Example 4

### Protein transduction of the chimeric proteins into eukaryotic cell lines.

The following cell-lines were used for the examples 4-11:

The defective cell-lines in the NHEJ repair pathway: the CHO lines: Xrss5 (KU80 ^{(-/-)}), Xr-1 (XRCC4^{(-/-)}), V3 (DNA-PKcs^{(-/-)}) and the corresponding parental cell-line AA8 (ATCC CRL1859) and also the cell-line: HIS P1.13-11 (KU70^{(-/-)}) and the corresponding parental cell-line CHO-K1 (ATCC CCL61). These lines wer grown in DMEM medium containing 10% fethal calf serum (FCS). Further were used the human glioma-cell-lines MO59J (DNA-PKcs ^{(-/-)}) and the corresponding parental lines M059K (wild type for DNA-PKcs) (Lees-Miller SP, et al. Science 1995, 267 (5201):1183-5) which were grown in DMEM/NUT.MIX-F12 1:1 medium with 10% FCS.

The line AT-5 is derived from an individual with defective ATM (ataxia-teleangectasia mutated protein) and as corresponding parental cell-line MRC-5 was used (Raj K, et al. Nature. 2001, 412 (6850): 914-7) both were cultivated in DMEM containing 10% FCS.

The neuroblastoma cell-lines IMR32 (ATCC CCL-127), GI-LIN and LAN-5 (Panarello C. et al. Cancer Genet. Cytogenet., 2000, 116:124-132) were grown in RPMI medium +Hepes 25mM containing essential amino acids and 10% FCS.

The human osteosarcoma cell line U20S (ATCC HTB96); the primary fibroblasts IMR90 (early passages) were grown in DMEM medium containing 10% FCS.

The colon carcinoma cell-line HCT-116 (defective for the mismatch repair gene human MLH1 protein (Raj K, et al. Nature. 2001, 412 (6850): 914-7)) was grown in McCoy's medium containing 10% FCS.

The transduction capacity of the chimeric proteins was assayed in U20S osteosarcoma cells and in the IMR90 primary fibroblasts cells. The cells were grown in DMEM containing 10% FCS and treated with the proteins of the invention in the same medium, in general in the presence of antibiotics. After 10 minutes of incubation of the cells with increasing quantities of the fusion protein SCPVUTAT (1, 10, 50, 100, 200 nM) and with the protein SCPVU (single chain PvuII without TAT) and SC34 as a control (prepared as described for SCPVUTAT except that the enzyme PvuII contains a mutant in position 34) cell extracts were prepared, separated on SDS-PAGE and assayed by immuno-blot analysis with a monospecific antibody to PvuII. Further, uptake was assayed after 30 min of incubation by immuno-fluorescence analysis using PvuII specific antibodies and nuclear costaining with propidium iodide. Together the data demonstrate, that the protein SCPVUTAT is enriched in the cells and in particular in the nucleus and uptake was in nearly 100% of the cells analysed as shown in Figure 2. In contrast, the fusion-protein containing no tat sequences (SCPVU) is not imported. These enrichments were not dependent on the denaturation of the protein, as the proteins used were soluble and prepared under native, non denaturing conditions. In fact, the proteins that were produced by the methods described and under native conditions were more effective in protein transduction and superior to the denaturation methods described recently for other proteins in the patent of Dowdy USP6,221,335.

### Example 5

Confirmation of the DSB induction after the treatment with the recombinant proteins of the invention in vivo by TUNEL assay.

After a brief treatment of the cells with SCPVUTAT the function of the proteins was assayed with terminal deoxynucleotidyl transferase (TdT) dUTP-FITC, in a TUNEL reaction (In Situ Cell Death Detection Kit, Roche Diagnostic, Mannheim Germany) which marks DSBs in vivo. The assay was done after fixation of the cells in 4% paraformaldehyde in PBS, 0.1% Triton. After the TUNEL reaction the cells are counter-stained with propidium J⁻. The use of derivatives impaired in the nuclease activity like the construct SC34 as a negative control, allows to estimate background activities. As short as 10 minutes after treatment with the fusion protein SCPVUTAT all of the cells tested showed (90-100%) TUNEL positive reaction. Among the cells tested are human or rodent cell lines including epithelial cells (HEK-293, MCF-7, HCT116 a colon carcinoma cell line that exhibits a defect in the mismatch repair gene hMLH1), primary fibroblasts (WI83, IMR90, Mouse Embryonic Fibroblast's, MEF). This confirms beside the efficient delivery of the fusion proteins of the invention to the nuclei of the cells exhibit endonucleolytic activity in vivo in all cell types assayed so far. In fact few minutes of treatment with SCPVUTAT and concentrations of the proteins in the cell culture supernatant as low as 10nM produce significant TUNEL activity in most of the cells, thus demonstrating direct and rapid functionality of the chimeric proteins in vivo. This aspect makes the system described in the invention by far more efficient as compared to endonucleases induced from transcriptional units or compared to any of the other type of protein transfection like electroporation, or precipitations with calcium phosphate or libid-derivatives i. e. lipofectin. This activity was cell cycle phase independent and in most of the cells was not correlated with apoptosis, as was demonstrated by the missing positiv markers for apoptosis (i.e. cytochrome C release or positive Anexin V staining).

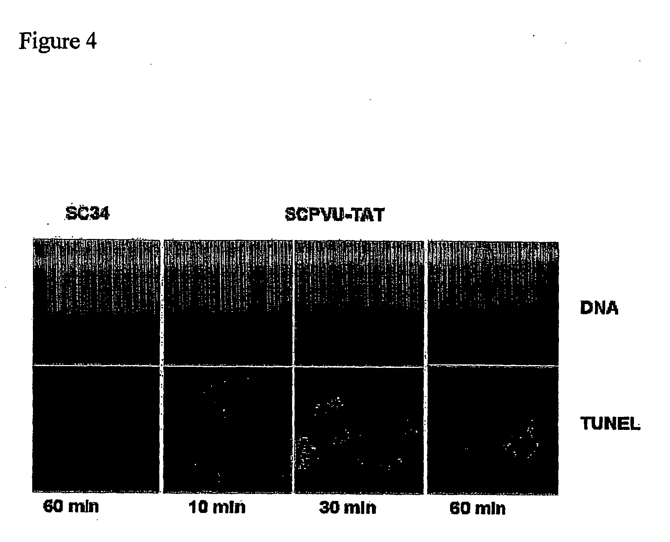

In Figure 4 results obtained from a TUNEL assay from U2OS cells after a treatment for 30 minutes with SCPVUTAT or SC34 are shown, these types of assays demonstrate the functionality of the SCPVUTAT construct.

### Example 6

### Evaluation of the effect of a treatment with the proteins of the invention on the cell cycle.

Finally, to confirm that the DSB's that are produced in vivo also induce perturbations in the cell cycle, DNA content in the various phases of the cell cycle was analysed by Fluorescence Activated Cell Sorting (FACS) after transduction of the proteins of the invention. After the treatment with diverse proteins at various concentrations and during different timepoints, the cells were fixed in 70% ethanol, treated with RNAse A and stained with propidium J⁻.

The FACS analysis was done using a FACSCalibur^{™} (Becton Dickinson) apparatus. The obtained data were evaluated with the CellQuest^{™} software program package. At least 3x10⁴ single events for every sample were analysed in the DDM mode. The statistic analysis of the DNA content distribution during the cell cycle was done with the MODFIT LT^{™} software program package. A 2N diploid DNA content correspond to cells in the G1 phase of the cell cycle, 4N represents a relative tetraploid DNA content and corresponds to G2 and/or M phase of the cell cycle. An intermediate DNA content represents S phase corresponding to a population actively replicating DNA during the cell cycle and a content that is below diploid 2N represents cells in apoptosis.

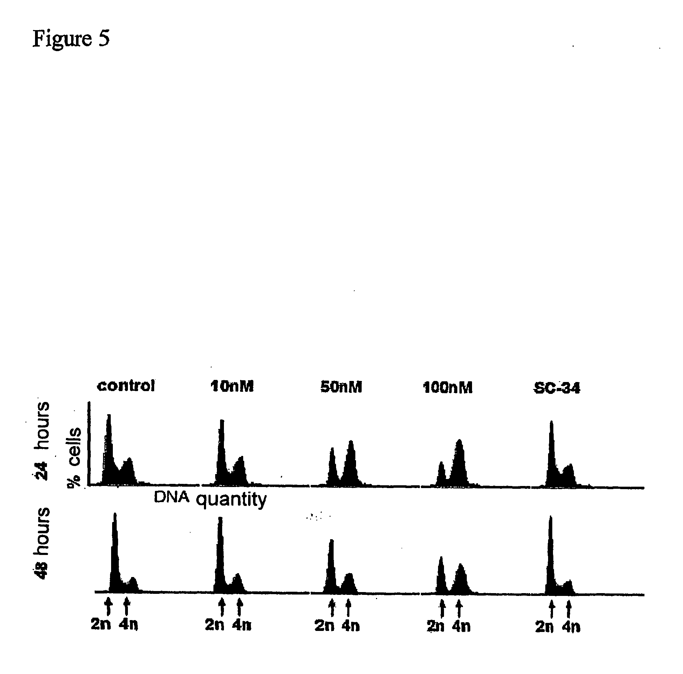

In Figure 5 is shown that an incubation for 24 hours with 10nM of the protein SCPVUTAT is sufficient induce an increase in the teraploid population of U20S cells. The dilution of the SCPVUTAT protein with fresh medium causes re-entry of the cells towards a normal cycling population during 24 hours, whereas successive addition of SCPVUTAT (every 12 hours i.e.) results in a stably induced cell cycle delay, in most cells that were analysed without any induction of apoptosis. Similar treatments with the proteins SC34 and SCPVU (with no TAT domain) do not show any change in the cell cycle distributions.

Other methods can help to finally discriminate between the G2 and M phase of a population detected as tetraploid in a FACS analysis (4N); i.e., in 4N DNA containing HCT116 and U20S cells that were treated with 100nM SCPVUTAT the nuclei remain big and do not show any mitotic structures or no nuclear envelope breakdown which indicates that cells remain in G2 and do not show any progression into M phase of the cell cycle and thus exhibit a G2 cell cycle block. Further biochemical analysis can be employed to demonstrate the kinase activity of the Cdc2-cyclin B complex. Moreover, the initiation of mitosis, i.e. induced with the microtuble blocking agents Nocodazol or Taxol, can be detected by the nuclear activity of the Cdc2 kinase and also by nuclear localisation of Cdc25C. These assays allow to dissect the exact point of cell cycle arrest after SCPVUTAT treatment and this further allows to detect disturbations in this phase of the cell cycle relative to a positive control. Similar experiments are well known in the art also for other phases of the cell cycle. The assay shown in Figure 7A and the immunofluorescence analysis shown in Figure 14 show examples for these types of experiments. The experiments as shown indicate that in the cell line HCT116 upon treatment with the fusions protein SCPVUTAT DSB's were induced and subsequent a reversible block in the phase of the cell cycle that corresponds to late S/G2.

### Example 7

### Effects of the proteins to cells with mutations in ATM/ATR.

The Ataxia telangiectasia mutated protein (ATM) and the Ataxia telangiectasia related mutated protein (ATR) represent the central signaling elements during checkpoint activation in response to DNA damage. These pathway coordinate cell cycle progression and the DNA repair machineries. These controls ensure the appropriate order of events in a case of a damage in a cell, i.e. cells do not progress into successive phases of the cell cycle before the DNA is repaired. This implicates controls in the cell cycle machinery by inhibition of cell cycle kinases and concomitant induction of negative regulators, among them tumour suppressors like p53 and their known transcriptional targets (i.e.: p21, 14-3-3 sigma), proteins that in turn induce a cell cycle block that is important for an exact function or for accurate fidelity of the effecttor proteins responsible for DNA repair. It is important to note, that mutations in many of these regulatory proteins show strong predispositions for cancer.

Treatment of U20S cells with 2mM caffeine, a metylxantin derivative (IC₅₀ < 1mM) induce a moderate delay in the G1 phase of the cell cycle and moreover the inhibitory effect sensitises the cells to DNA damage (Sarkaria et al., 1999). It was shown that caffeine inhibits the catalytic activity of at least three members of the class of the phospho-inositol 3 kinase (PI3) with all of them containing a homologous serine/threonine kinase region (PIKK), ATM,ATR and TOR. U20S cells that were grown for 30 hours in medium with 100nM SCPVUTAT in combination with 2mM caffeine do not exhibit the characteristic cell cycle stop of SCPVUTAT, but however induce a moderate G1 delay. This demonstrates that caffeine is antagonist to the effect of SCPVUTAT in vivo. Final experiments demonstrate that caffeine neither inhibits SCPVUTAT in vitro, nor does it influence the transducion capacity of the protein. The same rational used for these experiments applies for a selection of antagonistic compositions to SCPVUTAT that are active "in vivo" and constitute general compositions or lead compounds, active as inhibitors of the ATM and/or ATM/ATR pathway. The specificity for ATM/ATR or for any other protein involved in this pathway can then be finally dissected by using appropriate mutant cell lines. Due to the immediate well characterized and monospecific activity of SCPVUTAT, these molecules can be advantageously used in this assays. The easy use of the presented invention allows application in these types of assays also in large scale, such as high-throughput in combination advantageously with the application of the combinatorial chemistry and/or combinatorial molecular biology.

In an other embodiment SCPVUTAT is used to monitor the events that depend on an activation of ATM/ATR. ATM functions as a serine/threonin kinase on many substrates involved in the checkpoint that is activated by DNA damage. The phosphorylation of the substrates can or cannot influence the activation of a checkpoint response. Substrates of ATM or of ATM dependent kinases known in vivo includes: Nbs1, SMC1 (Structural Maintenance of Chromosomes), MDC1 (Mediator of DNA Damage), H2AX (Histone 2Ax), p53.

The in vivo induction of ATM/ATR on the substrate of p53-Ser15 in response to SCPVUTAT was analysed. The phosphorylation of p53 on Ser¹⁵ regulates protein stability and transcriptional activity of p53 and thus is essential for the transcriptional response of p53.

P53 phosphorylation on Ser15 in response to SCPVUTAT treatment was assayed in ATM positive or negative cell lines (MRC-5 and AT-5 respectively). For comparison parallel experiments were done with hydroxyurea (HU). HU functions as an inhibitor of the ribonucleotidyl-reductase and causes a stalling of the DNA replication fork during S phase and causes DSB's. For this immunoblotting experiments were done with extracts after treatment, by using specific antibodies to a phosphorylated Ser15 in p53 for specific detection. As shown in Figure 7, specific phosphorylation of p53-Ser¹⁵ is obtained after treatment with SCPVUTAT in ATM plus cell lines MRC-5 (Figure 7B) as well as for the ATM minus cell lines AT-5. The same was found for a treatment with HU that induces p53-Ser¹⁵ phosphorylation in a comparable however consistently higher fashion. This in vivo phosphorylation is sensitive to the ATM/ATR inhibitor caffeine; AT-5 cells incubated with 2mM caffeine, and are SCPVUTAT or HU treated do not exhibit significant p53-Ser¹⁵ phosphorylation (Figure 7C). As another example for a phosphorylation of a substrate of ATM in response to the object of the invention, histone H2AX Ser¹³⁹ phosphorylation was analysed. It was shown that ATM phosphorylates histone H2AX Ser¹³⁹ as an immediate response to DNA damage induced by radiation during S and G2 phase of the cell cycle. U20S cells were treated for 60 minutes with SCPVUTAT, fixed and analysed for histone H2AX Ser¹³⁹ phosphorylation. Cells were analysed with a specific antiserum to phospho-Ser 139 on histone H2AX and analysed in the microscope on a single cell basis. As a control histone 2B was analysed. The results demonstrate that treatment with SCPVUTAT rapidly induces histone H2AX Ser¹³⁹ phosphorylation and this activity can be detected in specific located in the nuclei of the treated cells but not of the non treated cells (Figure 12). Moreover the ATM kinase inhibitor Wortmanin (50µM) is antagonist for this reaction. The concentration of Wortmanin used are specific for ATM but do not inhibit ATR, thus represent a simple assay to distinguish between these two kinases. These types of experiments, where substrates of the ATM pathway are analysed help to define the biochemical and molecular targets induced by DSB's induced by SCPVUTAT. The biochemical and/or immunological analysis of the substrates of the ATM kinase by western-blotting , immun-fluorescence analysis, or enzymatic assays in cells or from cell extracts treated with the presented invention is useful for an interpretation of the state of a response of a checkpoint in vivo in a particular cell in comparison to a normal induction of the respective pathway.

Moreover, these assays can be advantageously used with the presented invention in automatic screens. The exact and well-defined nature of the damage induced in the DNA by the presented invention, makes the presented superior in respect to any other compound previously used in similar types of assays. The use of the presented invention in single assays or high-through-put screens has little need to consider secondary or pleotropic effects, an essential prerequisite for the success of a complex screen. The data obtained in these types of experiments can be considered as equivalent to the effects induced by a DSB. Moreover, the availability of mutated protein derivatives that do not exhibit nuclease activity like SC34 warrant important controls for these assays.

### Example 8

### Dependence of the SCPVUTAT induced effect from ATM and p53.

In general in the case of DNA damage mutations in the components of the corresponding mechanism of control (checkpoint) exhibit changes in the arrest-characteristics of the cell cycle and many of these mutations determine predisposition to cancer.

In contrast proteins that are involved as effector molecules for DNA repair in general exhibit normal arrest characteristics of the cell cycle and induce tumour progression mainly only in combination with defects in checkpoint components and show frequently synergism in a predisposition for tumours. Mutations in the effector molecules exhibit particular hypersensitivity towards DSB's, a characteristics that is significantly less pronounced in the case of checkpoint mutations. Due to the fact that the proteins of the invention are monospecific it is possible to interpret sensibility to DSB and the effects on the cell cycle as simple consequence of the induced damage. In fact it is possible to evaluate if the defects are in components of the control mechanisms (checkpoints) or in the repair machinery.

The cell cycle response to DSB's induced by SCPVUTAT treatment were analysed in p53 positive or negative cells. Whereas cells that are positive for p53 show cell cycle arrest behaviour in G1 and G2 with low S-phase values, but cells negative for p53 do not exhibit arrest characteristics of the cell cycle. Moreover this type of result was also obtained for the p53 downstream elements were mutations in p21 exhibit comparable behaviour to p53 minus cells and mutations in 14-3-3 sigma exhibit a G1 arrest confirming that this protein is partial overlapping with p53 and has its role only in the control of the G2 phase of the cell cycle. As finally demonstrated in Figure 8A, the mutants AT-5 were analysed after treatment with SCPVUTAT. Incubation for 36 hours of the cell lines mutated for ATM (AT-5) with SCPVUTAT results in a transient retardation of the cell cycle, as was determined by a DNA content FACS analysis. AT-5 cells show a strong increase in 4N G2 DNA content and do not show any G1 delay, and this indicates a complex defect in the G1 checkpoint (Figure 8). Significantly, the delay in G2 is not released and re-entry into the cell cycle is not observed after 24 hours in contrary to ATM positive cell lines (see Figure 5). A single addition of SCPVUTAT for 60 minutes to the AT-5 cells was sufficient to induce cell cycle stop after 24hours (Figure 8A) and finally leads to cell death, obtained from not correctly ligated DNA, and consequently, the clonogenic activity after a single treatment with SCPVUTAT for 60 minutes was below 0.02% for AT-5 cells. In these assays, the ATM positive control cell line MRC-5 was less sensitive to a SCPVUTAT treatment (Figure 8B). In addition, the cells were incubated with adriamycin as a control. This effects were due to the endonuclease activity of SCPVUTAT because colonies were formed with comparable efficiency to no treatment upon treatment with mutated nuclease versions. These examples demonstrate that various mutated cells show distinct cell cycle profiles in response to SCPVUTAT treatment and that this characteristic can be used for the diagnosis of cell cycle defects upon DSB' S.

### Example 9

### Evaluation of the diagnostic capacity of the protein SCPVUTAT on cells defective in the NHEJ (Non-homologous End-Joining) repair pathway.

NHEJ represents the principal mechanisms for a double strand DNA break repair in higher eucaryots in contrary to lower eucaryots where a repair by homologous recombination is more present. The proteins involved in this system are also involved in the final phases of the V(D)J immunglobulin recombination. In order to confirm if the proteins of the invention activate also the enzymes of the NHEJ in a specific matter, rodent cell lines containing individual mutations in one of the essential factors of this pathway were analysed.

In a colony formation assay summarized in Figure 9 and Figure 13 confirms that a single treatment with the protein of the invention SCPVUTAT is sufficient to strongly reduce colony formation activity in all the NHEJ mutated cell lines analysed.

To demonstrate the specificity of the repair of DSB's of the presented invention, rodent cells were used that contain homozygote loss of function mutations in one of the essential factors involved in the NHEJ repair for DSB's: the PIKK subunit catalytic domain containing regulatory subunit of the DNA dependent protein kinase (DNA-PKcs ; V3 cell lines, corresponding parental cell line AA8), the regulatory subunit of the DNA dependent protein kinase Ku70 (HIS P1. 13-11; corresponding parental cell line CHO-K1), the subunit Ku80 (xrss5; corresponding parental cell line AA8), and the DNA ligaseIV regulatory subunit XRCC4 (XR-1 cell lines; corresponding parental cell line AA8). These cell lines were analysed in the nuclease activity assays with SCPVUTAT in vivo and compared to the parental cell lines as indicated. Moreover these NHEJ mutant cells were tested with SCPVUTAT in clonogenic, colony-outgrowth assays that were assayed after a single addition of SCPVUTAT for 12 hours at a concentration of 10nM (grey bars) or at a concentration of 75nM (black bars) or without protein treatment (not treated, white bars). Seven to ten days after addition, cell growth was analysed by staining withGIEMSA-blue and quantification of diverse cell-concentrations. The results obtained from a representative experiment are summarized in Figure 9. Cells that are mutant for the subunit XRCC4 of the DNA LigaseIV are hypersensitive to blunt end DSB's induced by SCPVUTAT and more interesting, all the cells that contain mutants in the DNA-PK assayed, including the mutations of the catalytic subunit, exhibit as well a strong reduction in the colony formation assay. In contrast to parental cells, to cells that contain known defects in the miss-match repair pathway, like HCT116 that exhibit a homozygous mutation in the gene coding for the hMLH1 protein, do not show any increase in sensibility to a treatment with SCPVUTAT in similar assays. In a further example human glioblastoma cell lines that are defective in DNA-PKcs (M0059J) were treated with SCPVUTAT and confronted to the parental cell lines M059K. In analogy, cell growth was quantified after a single treatment with SCPVUTAT for 12 hours (10nM, grey bars; 75nM, black bars; not treated, white bars). Again, also the human DNA-PKcs mutant cells show a significant differential increase in sensibility in response to SCPVUTAT treatment, Figure 13A, B). These cells were plated into multiwell-microtiter-plates at different concentrations and treated with SCPVUTAT as indicated and stained with GIEMSA-blu. The multiwell-microtiter-plates (for an example see Figure 13A) were scaned using the VersaDOC^{R} (BioRad) imaging system and growth was automatically quantified with the software package Quantify One^{R} for the analysis of the microtiter-plates (microtiter-plates scanning software). The data obtained were further summarized in a histogram as outlined in Figure 13. Consistent with the results obtained from the rodent cell lines that are mutated for DNA-PKcs , also the human cell lines demonstrated hypersensitivity upon SCPVUTAT treatment, and this confirms the high specificity of SCPVUTAT for the introduction of DSB's. These experiments demonstrate the feasibility of the used system including applications in semi- or total automatic assays.

### Example 10

### Assay for the screening of candidate compounds as synergist or antagonist to stress induced by DSB.

U20S cells were incubated with 10nM SCPVUTAT and with 10µM H₂O₂ alone or in combination ant the cell cycle profile was analysed by FACS. Simultaneous incubation of the cells with the two compounds caused a differential, highly increased change of the cell cycle profile with a strong increase of the corresponding G2 phase peak from 20% to 45% (Figure 11A, compared to Figure 11D) in comparison with no increase in G2 with 10µM H₂O₂ alone (Figure 11C) and with a minor increase of 20% to 28% in G2 (Figure 11A, compared to Figure 11B) in the case of 10nM SCPVUTAT alone. Based on the results from the incubations with the single components, the incubation with both components simultaneously clearly exhibits a synergistic effect for a perturbation of the cell cycle.

In a successive, similar experiment were the SCPVUTAT protein was incubated together with aphidicolin (a DNA intercalating agent and topoisomerase II inhibitor), no synergistic effect was observed and both components show only additive behaviour. This example, again demonstrates the simplicity of the assay, that can be used for detection of synergism or antagonism to the cellular stress that is induced by blunt end DSB from the proteins of the invention. As outlined above, the advantageous of the assay comes from the monospecific DNA damage induced, the possibility to measure the background of the system with the nuclease impaired protein SC34 and the extremely fast and easy use of the assay in virtually any cell and any phase of the cell cycle. This was not described before for any other reagent capable to induce DSB's in vivo. Thus this type of assay shown for radicals and caffeine (Figure 7C), can be used for every type of screen for compositions that interfere with the DNA repair pathway and controls thereof.

### Example 11

### Induction of apoptosis in neuroblastoma from treatment with SCPVUTAT

Whereas many of the analysed cells of epithelial as well as mesenchymal origin did not show apoptosis upon treatment with SCPVUTAT, in some neuroblasoma cell lines (IMR32, LAN5, GI-LIN,) but also various primary neuroblastoma cell isolates from the clinics a significant amount of apoptosis was induced. Figure 10 demonstrates an example of an induction of apoptosis by the proteins of the invention in these cells. For the analysis the DNA content was assayed with FACS after staining of the cells with propidium J⁻. Apoptotic cells (A) were detected as the cells exhibiting a DNA content below 2N. The calculated regions are indicated on top of each histogram with arrows for the corresponding areas. Whereas 10nM of SCPVUTAT rapidly induces 18% apoptotic cells in 30 hours (Figure 10B), and the addition of 100nM of SCPVUTAT gives the induction of more than 29% of apoptotic cells in the same time period (Figure 10C), contrary, the nuclease impaired version SC34 does not induce this effect. These experiments demonstrate a highly specific induction of apoptosis in neuroblastoma cells, and the proteins of the invention apply as prime candidates for a therapeutic treatment of these types of tumours.

Moreover in combination with tissue specific delivery systems, the sequences that are object of the presented invention can contain sufficient specificity as candidate substances for the therapy of neuroblastoma. In fact, in turn it is a valid implication that the invention presented applies as a platform-technology for a screen for tissue specific delivery sequences.

In addition, like already anticipated in previous examples (Example 8), the presented invention can also be used to search for specific lead compounds or for specific combinations of molecules that are able to induce specific, differential apoptosis in target cells, advantageously in malignant cells from tumours but not, or to a much lower extend in normal cells of the same individual.

### Example 12

### Cellular localisation of cell cycle proteins after treatment with SCPVUTAT by immuno-microscopic analysis.

HCT116 (p53(+/+)) not treated, or treated for 30 hours with SCPVUTAT (100nM), or with Taxol (0.2µg/ml) were fixated with 3% PFA and treated with specific antibodies to cyclin B1 or for Cd-c25C. The immunofluorescence was analysed with the microscope (see Figure 14) with specific primary antibodies to cyclin B1 conjugated to FITC labelled secondary antibodies (green) or with specific primary antibodies to Cdc25C conjugated to TRITC labelled secondary antibodies (red). The cytoplasmatic distribution of the cell cycle markers Cdc25C and Cyclin B obtained from cells with a 4N DNA content after treatment with SCPVUTAT demonstrate a G2 phase stop and that the treated cells do not proceed into M phase of the cell cycle.

This result is different from the results obtained with Taxol were the prevalent nuclear localization indicates a stop in M phase of the cell cycle.

The change of the special distribution of the two marker proteins - i.e. cytoplasmatic, corresponding to an inactive cyclinB kinase, in contrast to a nuclear localisation that corresponds to an active cyclinB kinase and a subsequent progression of the cell cycle into anaphase - and a successive activation of the cdc2/cyclin B kinase by dephosphorylation with the phosphatase Cdc25C (after release of the 14-3-3 protein from Cdc25C and subsequent activation of the phosphatase), constitutes a hallmark for the G2/M transition. In fact this pathway substitutes the rate-limiting step in this phase of the cell cycle.

## Claims

1. A chimeric polypeptide capable to penetrate biological membranes comprising:
a. a polypeptide exhibiting affinity for specific nucleotide sequences
b. a DNA modifying enzyme,
c. a region with a cellular membrane-crossing delivery activity,
wherein the DNA modifying enzyme is a restriction endonuclease from the type II class or their subunits or functional fragments and regions a., b., and c. are covalently linked to each other.

2. A polypeptide according to claim 1 wherein the region with cellular membrane-crossing delivery activity is selected from the group consisting of peptides and polypeptides.

3. A polypeptide according to claim 1 wherein the activity of polypeptide a and polypeptide b. are contained in the same molecule.

4. A polypeptide according to claims 1 and 3 wherein the polypeptide with the specific nucleotide binding activity and the polypeptide that contains the enzymatic DNA modifying activity are restriction endonucleases or their subunits or functional fragments.

5. A polypeptide according to any one of claims 1 to 4 wherein the restriction endonuclease is selected from the group consisting of EcoRV, PvuII, HinfI and their subunits and functional fragments.

6. A polypeptide according to any one of claims 1 to 5 wherein the endonuclease activity is modified but the nucleic acid binding specificity is comparable to the native enzyme.

7. A polypeptide according to any one of claims 1 to 6 wherein the region for cellular membrane-crossing delivery activity comprises at least one peptide selected from the group consisting of VP22 of Herpes Simplex Virus, Tat of HIV-1, Rev of Hiv-1, the Antennapedia homeodomain and fragments there of.

8. A polypeptide according to claim 7 derived from the HIV-1 Tat protein comprising the peptide: YGRKKRRQRRR or point mutations or functional mutations thereof.

9. A polypeptide according to any one of claims 4 to 8 wherein the polypeptide equipped with specific nucleotide binding affinity and the DNA modifying enzyme are represented by a single polypeptide coding for a classII restriction-endonuclease chosen from EcoRV, PvuII, and HinfI or their subunits or functional fragments and the cellular membrane-crossing delivery function is a peptide contained in the amino acid sequence of HIV-1 tat.

10. A polypeptide according to claim 9 wherein the subunits of the restriction endonuclease are covalently connected as a single chain protein.

11. A polypeptide according to claim 10 comprising SEQ ID No. 2.

12. A chimeric polypeptide according to any one of claims 1 to 11 containing non natural amino acids.

13. An isolated polynucleotide coding for a polypeptide comprised in any one of claims 1 to 11 and comprising SEQ ID No. 1.

14. A vector comprising the polynucleotide sequence according to claim 13.

15. Host cells that are transformed with the vectors according to claim 14.

16. A polypeptide according to any one of claims 1 to 12, polynucleotides according to claim 13, vectors according to claim 14, cells according to claim 15 for a therapeutic use.

17. Pharmaceutical compositions containing the polypeptides according to any one of claims 1 to 12 as active principle, in combination with suitable excipents, emulsifiers or diluents.

18. The use of the chimeric molecules, the polynucleotides, the vectors, the cells according to claim 16 for the preparation of pharmaceuticals for the prevention, the cure or the diagnosis of neoplastic disease or the predisposition for this disease.

19. A method for the modification at specific sites in the genome of an isolated cell comprising essentially the treatment of these cells with a chimeric polypeptide according to any one of claims 1 to 12.

20. A method according to claim 19 based on the use of the chimeric polypeptides according to any one of claims 1 to 12 and wherein the introduced modifications at specific sites in the genome of isolated cells are DNA double strand breaks.

21. A method for screening for compositions that are able to modulate the genome-repair activities or to modulate cell cycle control and checkpoint activities in a cellular system that contains essentially the following:
i) incubation of the isolated cells with the chimeric polypeptides according to any of claims 1 to 12,
ii) addition of compositions or collections of compositions optional in the presence of radical producing substances,
iii) characterisation and/or measurement of the cellular response.

22. A method for the activation of the DNA repair and checkpoint mechanisms in vitro by the use of the procedure according to the claims 19 and 20 or the use of the chimeric polypeptides according to any one of claims 1 to 12.

23. A method for the diagnosis of a genetic defect in the DNA repair and cell cycle control and checkpoint pathways in vitro, comprising essentially the following steps:
a) optional cultivation of isolated cells of a test sample,
b) incubation of these cells with the chimeric polypeptides according to any one of claims 1 to 12, optional in parallel with suited reference cell-lines,
c) caracterisation and/or measurement of the cellular responses.

24. A method according to the claims 21 and 23 wherein respectively at point iii) and c) the cellular response is chosen between: clonogenic activity, phosphorylation state or expression level of biochemical marker consisting of intracellular proteins, the localisations of those proteins on the nuclear or cytoplasmatic level, the total DNA content of the cell population, the cellular proliferation.

25. A method according to claim 24 wherein those intracellular proteins are selected from the group consisting of p53, ATM, Chk1, Chk2, BRCA-1, BRCA-2, Nbs1, Mre11, Rad50, Rad51 and histones.

26. A method according to claim 24 and 25 for the in vitro diagnosis of a genetic predisposition for tumours or for diagnosis for radiation sensitivity.

27. The use of the chimeric molecules according to any one of claims 1 to 12 for the introduction of a cell cycle block in isolated cells.

28. The use of the chimeric molecules according to any one of claims 1 to 12 for the the induction of apoptosis in isolated cells from neuroblastoma origin.

29. A kit for the diagnosis of a genetic defect in the DNA repair system or in the control system for the cell cycle (check-points) in an isolated cell containing the chimeric polypeptide according to any one of claims 1 to 12 in combination with a tube containing a chimeric polypeptide for the control wherein the polypeptide exhibits specific DNA binding activity but is impaired in nuclease activity.

30. A kit according to claim 29 wherein the chimeric polypeptide according to any one of claims 1 to 12 has SEQ ID No. 2 and the polypeptide for the control is the polypeptide SC34.

31. A kit according to claim 30 for the in vitro diagnosis of genetic defects that determine predisposition for tumours in isolated cells.

32. A kit according to claim 31 for the diagnosis of the radio-sensitivity of a tumour.

## Patentansprüche

1. Ein chimäres Polypeptid, das zum Penetrieren biologischer Membranen fähig ist, umfassend:
a) ein Polypeptid, das eine Affinität zu spezifischen Nukleotid-Sequenzen aufweist
b) ein DNA-modifizierendes Enzym
c) einen Bereich mit einer die Zellmembran durchquerende Übermbringeraktivität,
wobei das DNA-modifizierende Enzym eine Restriktionsendonuklease der Typ-II-Klasse oder ihre Untereinheiten oder funktionellen Fragmente und Regionen a), b) und c) miteinander kovalent verbunden sind.

2. Ein Polypeptid nach Anspruch 1, wobei die Region mit der die Zellmembran durchquerenden Aktivität ausgewählt ist aus der Gruppe bestehend aus Peptiden und Polypeptiden.

3. Ein Polypeptid nach Anspruch 1, wobei die Aktivität des Polypeptids a) und Polypeptids b) im gleichen Molekül enthalten sind.

4. Ein Polypeptid nach Anspruch 1 und 3, wobei das Polypeptid mit der spezifischen Nukleotid-bindenden Aktivität und das Polypeptid enthaltend die enzymatische DNA-modifizierende Aktivität Restriktionsendonukleasen oder ihre Untereinheiten oder funktionellen Fragmente sind.

5. Ein Polypeptid nach einem der Ansprüche 1 bis 4, wobei die Restriktionsendonuklease ausgewählt ist aus der Gruppe bestehend aus EcoRV, PvuII, HinfI und ihren Untereinheiten und funktionellen Fragmenten.

6. Ein Polypeptid nach einem der Ansprüche 1 bis 5, wobei die Aktivität der Endonuklease modifiziert ist, die Nukleinsäurebindende Spezifität jedoch mit der des nativen Enzyms vergleichbar ist.

7. Ein Polypeptid nach einem der Ansprüche 1 bis 6, wobei die Region für die die Zellmembran durchquerende Oberbringeraktivität zumindest ein Peptid umfasst, welches ausgewählt ist aus der Gruppe bestehend aus VP22 vom Herpes-Simplex-Virus, Tat von HIV-1, Rev von Hiv-1, der Antennapedia Homeodomäne und Fragmenten davon.

8. Ein Polypeptid nach Anspruch 7, welches vom HIV-1-Tat-Protein abgeleitet wurde, umfassend das Peptid: YGRKKRRQRRR oder Punktmutuationen oder funktionelle Mutationen davon.

9. Ein Polypeptid nach einem der Ansprüche 4 bis 8, wobei das mit der spezifischen Nukleotid-bindenden Affinität ausgestattete Polypeptid und das DNA-modifizierende Enzym durch ein einzelnes Polypeptid repräsentiert sind, das für eine Restriktionsendonuklease der Klasse II ausgewählt aus EcoRV, PvuII und Hinfl oder ihren Untereinheiten oder funktionellen Fragmenten kodiert, und es sich bei der die Zellmembran durchquerende Überbringerfunktion um ein Peptid handelt, welches in der Aminosäuresequenz von HIV-1 tat enthalten ist.

10. Ein Polypeptid nach Anspruch 9, wobei die Untereinheiten der Restriktionsendonuklease als einkettiges Polypeptid kovalent verbunden sind.

11. Ein Polypeptid nach Anspruch 10, umfassend SEQ ID Nr. 2.

12. Ein chimäres Polypeptid nach einem der Ansprüche 1 bis 11, enthaltend nicht natürliche Aminosäuren.

13. Ein isoliertes Polynukleotid, kodierend für ein Polypeptid, das in einem der Ansprüche 1 bis 11 inkludiert ist, und umfassend SEQ ID Nr. 1.

14. Ein Vektor umfassend die Polynukleotid-Sequenz nach Anspruch 13.

15. Wirtszellen, die mit dem Vektor gemäß Anspruch 14 transformiert sind.

16. Ein Polypeptid nach einem der Ansprüche 1 bis 12, Polynukleotide nach Anspruch 13, Vektoren nach Anspruch 14, Zellen nach Anspruch 15 zur therapeutischen Verwendung.

17. Pharmazeutische Zusammensetzungen enthaltend die Polypeptide nach einem der Ansprüche 1 bis 12 als aktiver Grundbestandteil, in Kombination mit geeigneten Hilfsstoffen, Emulgatoren oder Verdünnungsmitteln.

18. Verwendung der chimären Moleküle, der Polynukleotide, der Vektoren, der Zellen gemäß Anspruch 16 zur Herstellung von Pharmazeutika zur Vorbeugung, Heilung oder Diagnose der neoplastischen Erkrankung oder Prädisposition für diese Erkrankung.

19. Ein Verfahren zur Mofifizierung an spezifischen Stellen im Genom einer isolierten Zelle, im Wesentlichen umfassend die Behandlung dieser Zellen mit einem chimären Polypeptid gemäß einem der Ansprüche 1 bis 12.

20. Ein Verfahren nach Anspruch 19, basierend auf der Verwendung des chimären Polypeptids gemäß einem der Ansprüche 1 bis 12 und wobei die eingeführten Modifikationen bei spezifischen Stellen im Genom der isolierten Zellen Doppelstrangunterbrechungen der DNA sind.

21. Ein Verfahren zum Screenen von Zusammensetzungen, die zur Modulation Genom-reparierender Aktivitäten oder der Zellzyklus-Regulation und Checkpoint-Aktivitäten in einem Zellsystem fähig sind, das im Wesentlichen Folgendes enthält:
i) Inkubation der isolierten Zellen mit den chimären Polypeptiden gemäß einem der Ansprüche 1 bis 12,
ii) Zugabe der Zusammensetzungen oder Sammlungen von Zusammensetzungen, wahlweise in Anwesenheit von radikal-produzierenden Substanzen,
iii) Charakterisierung und/oder Messung der Zellantwort.

22. Ein Verfahren zur Aktivierung der DNA-Reparatur und der Checkpoint-Mechanismen in vitro unter Verwendung der Arbeitsschritte gemäß den Ansprüchen 19 und 20 oder Verwendung der chimären Polypeptide nach einem der Ansprüche 1 bis 12.

23. Ein Verfahren zur Diagnose eines Gendefekts in der DNA-Reparatur und Zellzyklus-Regulation und Checkpoint-Wege in vitro, im Wesentlichen umfassend die folgenden Schritte:
a) wahlweise Kultivierung der isolierten Zellen einer Testprobe,
b) Inkubation dieser Zellen mit den chimären Polypeptiden gemäß einem der Ansprüche 1 bis 12, wahlweise parallel mit den geeigneten Referenz-Zelllinien,
c) Charakterisierung und/oder Messung der Zellantworten.

24. Ein Verfahren nach den Ansprüchen 21 und 23, wobei bei Punkt iii) bzw. c) die Zellantwort ausgewählt ist aus: klonogener Aktivität, Status der Phosphorylierung oder Expressionslevel der biochemischen Marker, bestehend aus intrazellulären Proteinen, der Lokalisierungen dieser Proteine auf nuklearer oder zytoplasmischer Ebene, dem DNA-Gesamtgehalt der Zellpopulation, der Zellproliferation.

25. Ein Verfahren nach Anspruch 24, wobei diese intrazellulären Proteine ausgewählt sind aus der Gruppe bestehend aus p53, ATM, Chk1, Chk2, BRCA-1, BRCA-2, Nbs1, Mre11, Rad50, Rad51 und Histone.

26. Ein Verfahren nach Anspruch 24 und 25 zur in-vitro-Diagnose einer genetischen Prädisposition für Tumore oder zur Diagnose für Strahlungsempfindlichkeit.

27. Die Verwendung von chimären Molekülen nach einem der Ansprüche 1 bis 12 zur Einführung eines Zellzyklus-Blocks in isolierte Zellen.

28. Die Verwendung von chimären Molekülen nach einem der Ansprüche 1 bis 12 zur Einführung von Apoptose in isolierte Zellen, die aus einem Neuroblastom stammen.

29. Ein Set zur Diagnose eines genetischen Defekts im DNA-Reparatur-System oder im Regulationssystem des Zellzyklus (Check-points) in einer isolierten Zelle, enthaltend das chimäre Polypeptid nach einem der Ansprüche 1 bis 12, in Kombination mit einem Schlauch enthaltend ein chimäres Polypeptid zur Regulation, wobei das Polypeptid eine spezifische DNA-bindende Aktivität aufweist, jedoch durch Nuklease-Aktivität beschädigt ist.

30. Ein Set nach Anspruch 29, wobei das chimäre Polypeptid nach einem der Ansprüche 1 bis 12 die SEQ ID Nr. 2 aufweist und das Polypeptid für die Regulation das Polypeptid SC34 ist.

31. Ein Set nach Anspruch 30 zur in-vitro-Diagnose eines genetischen Defekts, das die Prädisposition für Tumore in isolierten Zellen bestimmt.

32. Ein Set nach Anspruch 31 zur Diagnose der Bestrahlungsempfindlichkeit eines Tumors.

## Revendications

1. Polypeptide chimérique capable de pénétrer des membranes biologiques comprenant :
a. un polypeptide présentant de l'affinité pour des séquences de nucléotides spécifiques,
b. une enzyme modifiant l'ADN,
c. une région ayant une activité de délivrance par traversée de la membrane cellulaire,
dans lequel l'enzyme modifiant l'ADN est une endonucléase de restriction du type enzymes de classe II, ou leurs sous-unités, ou des fragments fonctionnels de celles-ci, et les régions a., b., et c. sont liées de manière covalente les unes aux autres.

2. Polypeptide selon la revendication 1, dans lequel la région ayant l'activité de délivrance par traversée de la membrane cellulaire est choisie dans le groupe constitué par des peptides et des polypeptides.

3. Polypeptide selon la revendication 1, dans lequel les activités du polypeptide a. et du polypeptide b. sont contenues dans la même molécule.

4. Polypeptide selon les revendications 1 et 3, dans lequel le polypeptide ayant l'activité de liaison à des nucléotides spécifiques et le polypeptide qui contient l'activité enzymatique modifiant l'ADN sont des endonucléases de restriction ou leurs sous-unités ou des fragments fonctionnels de celles-ci.

5. Polypeptide selon l'une quelconque des revendications 1 à 4, dans lequel l'endonucléase de restriction est choisie dans le groupe constitué par EcoRV, PvuII, HinfI, et leurs sous-unités et des fragments fonctionnels de celles-ci.

6. Polypeptide selon l'une quelconque des revendications 1 à 5, dans lequel l'activité de l'endonucléase est modifiée mais la spécificité de liaison à l'acide nucléique est comparable à celle de l'enzyme native.

7. Polypeptide selon l'une quelconque des revendications 1 à 6, dans lequel la région pour l'activité de délivrance par traversée de la membrane cellulaire comprend au moins un peptide choisi dans le groupe constitué par VP22 du virus de l'Herpes simplex, Tat du VIH-1, Rev du VIH-1, l'homéodomaine Antennapedia et des fragments de celles-ci (celui-ci).

8. Polypeptide selon la revendication 7, dérivé de la protéine Tat du VIH-1, comprenant le peptide : YGRKKRRQRRR ou des mutations ponctuelles ou des mutations fonctionnelles de celui-ci.

9. Polypeptide selon l'une quelconque des revendications 4 à 8, dans lequel le polypeptide doté d'une affinité de liaison à des nucléotides spécifiques et l'enzyme modifiant l'ADN sont représentés par un polypeptide unique codant pour une endonucléase de restriction de classe II choisie parmi EcoRV, PvuII et HinfI, ou leurs sous-unités ou des fragments fonctionnels de celles-ci et la fonction de délivrance par traversée de la membrane cellulaire consiste en un peptide contenu dans la séquence d'aminoacides de Tat du VIH-1.

10. Polypeptide selon la revendication 9, dans lequel les sous-unités de l'endonucléase de restriction sont reliées de manière covalente sous la forme d'une protéine à chaîne unique.

11. Polypeptide selon la revendication 10, comprenant SEQ ID No. 2.

12. Polypeptide chimérique selon l'une quelconque des revendications 1 à 11, contenant des aminoacides non naturels.

13. Polynucléotide isolé codant pour un polypeptide présent selon l'une quelconque des revendications 1 à 11 et comprenant SEQ ID No. 1.

14. Vecteur comprenant la séquence du polynucléotide selon la revendication 13.

15. Cellules hôtes qui sont transformées avec les vecteurs selon la revendication 14.

16. Polypeptide selon l'une quelconque des revendications 1 à 12, polynucléotides selon la revendication 13, vecteurs selon la revendication 14, cellules selon la revendication 15, destinés à une utilisation thérapeutique.

17. Compositions pharmaceutiques contenant les polypeptides selon l'une quelconque des revendications 1 à 12 en tant que principe actif, en association avec des excipients, des émulsifiants ou des diluants appropriés.

18. Utilisation des molécules chimériques, des polynucléotides, des vecteurs, des cellules selon la revendication 16, pour la préparation de produits pharmaceutiques destinés à la prévention, à la guérison ou au diagnostic d'une maladie néoplasique ou de la prédisposition à cette maladie.

19. Procédé pour la modification au niveau de sites spécifiques dans le génome d'une cellule isolée comprenant essentiellement le traitement de ces cellules avec un polypeptide chimérique selon l'une quelconque des revendications 1 à 12.

20. Procédé selon la revendication 19, basé sur l'utilisation des polypeptides chimériques selon l'une quelconque des revendications 1 à 12 et dans lequel les modifications introduites au niveau de sites spécifiques dans le génome de cellules isolées représentent des cassures du double brin d'ADN.

21. Procédé pour le criblage de compositions qui sont capables de moduler les activités de réparation du génome ou de moduler le contrôle du cycle cellulaire et les activités de (postes de) contrôle d'un système cellulaire, qui contient essentiellement ce qui suit :
i) l'incubation des cellules isolées avec les polypeptides chimériques selon l'une quelconque des revendications 1 à 12,
ii) l'addition de compositions ou de collections de compositions éventuelles en présence de substances produisant des radicaux,
iii) la caractérisation et/ou la mesure de la réponse cellulaire.

22. Procédé pour l'activation de la réparation de l'ADN et des mécanismes de contrôle in vitro par l'utilisation du mode opératoire selon les revendications 19 et 20 ou l'utilisation des polypeptides chimériques selon l'une quelconque des revendications 1 à 12.

23. Procédé pour le diagnostic d'un défaut génétique dans la réparation de l'ADN et le contrôle du cycle cellulaire et les voies de contrôle in vitro, comprenant essentiellement les étapes suivantes :
a) la culture éventuelle de cellules isolées d'un échantillon à tester,
b) l'incubation de ces cellules avec les polypeptides chimériques selon l'une quelconque des revendications 1 à 12, éventuellement en parallèle avec des lignées de cellules de référence appropriées,
c) la caractérisation et/ou la mesure des réponses cellulaires.

24. Procédé selon les revendications 21 et 23, dans lequel, respectivement aux points iii) et c), la réponse cellulaire est choisie parmi : une activité clonogénique, un état de phosphorylation ou un taux d'expression de marqueur biochimique consistant en protéines intracellulaires, les situations de ces protéines au niveau nucléaire ou cytoplasmique, la teneur en ADN total de la population de cellules, la prolifération cellulaire.

25. Procédé selon la revendication 24, dans lequel ces protéines intracellulaires sont choisies dans le groupe constitué par p53, ATM, Chk1, Chk2, BRCA-1, BRCA-2, Nbs1, Mre11, Rad50, Rad51 et des histones.

26. Procédé selon la revendication 24 et 25, pour le diagnostic in vitro d'une prédisposition génétique aux tumeurs ou pour le diagnostic d'une sensibilité au rayonnement.

27. Utilisation des molécules chimériques selon l'une quelconque des revendications 1 à 12, pour l'introduction d'un blocage du cycle cellulaire dans des cellules isolées.

28. Utilisation des molécules chimériques selon l'une quelconque des revendications 1 à 12 pour l'induction d'apoptose dans des cellules isolées provenant d'un neuroblastome.

29. Trousse pour le diagnostic d'un défaut génétique dans le système de réparation de l'ADN ou dans le système de contrôle du cycle cellulaire (postes de contrôle) dans une cellule isolée contenant le polypeptide chimérique selon l'une quelconque des revendications 1 à 12 en association avec un tube contenant un polypeptide chimérique pour le témoin, dans laquelle le polypeptide présente une activité de liaison spécifique à l'ADN mais est altéré en activité nucléase.

30. Trousse selon la revendication 29, dans laquelle le polypeptide chimérique selon l'une quelconque des revendications 1 à 12 possède la SEQ ID No. 2 et le polypeptide pour le témoin est le polypeptide SC34.

31. Trousse selon la revendication 30, pour le diagnostic in vitro de défauts génétiques qui déterminent une prédisposition aux tumeurs dans des cellules isolées.

32. Trousse selon la revendication 31, pour le diagnostic de la radiosensibilité d'une tumeur.


### Sequence listing

<110> International Center for Genetic Engineering and Biotechnology (ICGEB)
<120> chimeric polypeptides and their use
<130> 3916
<160> 4
<170> Patent In version 3.1
<210> 1
<211> 1020
<212> DNA
<213> Proteus vulgaris
<220>
   <221> CDS
   <222> (1)..(1020)
   <223> 1...471: PvuII 472...483: linker 484...951: PvuII 952...999: TAT 1000...1020: 6 his
<400> 1
   atg agc cac cca gat cta aat aaa tta tta gag ctt tgg ccg cat ata      48
   Met Ser His Pro Asp Leu Asn Lys Leu Leu Glu Leu Trp Pro His Ile
   1               5                   10                  15
   cag gaa tat caa gac tta gca tta aaa cat gga ata aat gat att ttt      96
   Gln Glu Tyr Gln Asp Leu Ala Leu Lys His Gly Ile Asn Asp Ile Phe
               20                  25                  30
   caa gat aat ggt gga aag ttg ctt caa gtc ctt cta att aca gga tta     144
   Gln Asp Asn Gly Gly Lys Leu Leu Gln Val Leu Leu Ile Thr Gly Leu
           35                  40                  45
   aca gta cta cca gga cga gaa ggt aat gat gct gta gat aac gca gga     192
   Thr Val Leu Pro Gly Arg Glu Gly Asn Asp Ala Val Asp Asn Ala Gly
       50                  55                  60
   caa gaa tac gag tta aaa tca ata aac ata gac ctc act aaa ggt ttt     240
   Gln Glu Tyr Glu Leu Lys Ser Ile Asn Ile Asp Leu Thr Lys Gly Phe
   65                  70                  75                  80
   tca act cac cac cac atg aat cct gta att att gca aaa tat aga caa     288
   Ser Thr His His His Met Asn Pro Val Ile Ile Ala Lys Tyr Arg Gln
                   85                  90                  95
   gta cct tgg att ttt gcc ata tac cgt ggt atc gca ata gaa gct ata     336
   Val Pro Trp Ile Phe Ala Ile Tyr Arg Gly Ile Ala Ile Glu Ala Ile
               100                 105                 110
   tac aga tta gag cca aaa gat cta gaa ttt tac tat gat aaa tgg gaa      384
   Tyr Arg Leu Glu Pro Lys Asp Leu Glu Phe Tyr Tyr Asp Lys Trp Glu
           115                 120                 125
   agg aaa tgg tat tca gat ggg cat aaa gat att aac aac cct aaa ata      432
   Arg Lys Trp Tyr Ser Asp Gly His Lys Asp Ile Asn Asn Pro Lys Ile
       130                 135                 140
   cct gta aaa tat gta atg gaa cat ggg aca aag att tac gga tcc gga      480
   Pro Val Lys Tyr Val Met Glu His Gly Thr Lys Ile Tyr Gly Ser Gly
   145                 150                 155                 160
   gga agt cac cca gat cta aat aaa tta tta gag ctt tgg ccg cat ata      528
   Gly Ser His Pro Asp Leu Asn Lys Leu Leu Glu Leu Trp Pro His Ile
                   165                 170                 175
   cag gaa tat caa gac tta gca tta aaa cat gga ata aat gat att ttt      576
   Gln Glu Tyr Gln Asp Leu Ala Leu Lys His Gly Ile Asn Asp Ile Phe
               180                 185                 190
   caa gat aat ggt gga aag ttg ctt caa gtc ctt cta att aca gga tta      624
   Gln Asp Asn Gly Gly Lys Leu Leu Gln Val Leu Leu Ile Thr Gly Leu
           195                 200                 205
   aca gta cta cca gga cga gaa ggt aat gat gct gta gat aac gca gga      672
   Thr Val Leu Pro Gly Arg Glu Gly Asn Asp Ala Val Asp Asn Ala Gly
       210                 215                 220
   caa gaa tac gag tta aaa tca ata aac ata gac ctc act aaa ggt ttt      720
   Gln Glu Tyr Glu Leu Lys Ser Ile Asn Ile Asp Leu Thr Lys Gly Phe
   225                 230                 235                 240
   tca act cac cac cac atg aat cct gta att att gca aaa tat aga caa      768
   Ser Thr His His His Met Asn Pro Val Ile Ile Ala Lys Tyr Arg Gln
                   245                 250                 255
   gta cct tgg att ttt gcc ata tac cgt ggt atc gca ata gaa gct ata      816
   Val Pro Trp Ile Phe Ala Ile Tyr Arg Gly Ile Ala Ile Glu Ala Ile
               260                 265                 270
   tac aga tta gag cca aaa gat cta gaa ttt tac tat gat aaa tgg gaa      864
   Tyr Arg Leu Glu Pro Lys Asp Leu Glu Phe Tyr Tyr Asp Lys Trp Glu
           275                 280                 285
   agg aaa tgg tat tca gat ggg cat aaa gat att aac aac cct aaa ata      912
   Arg Lys Trp Tyr Ser Asp Gly His Lys Asp Ile Asn Asn Pro Lys Ile
       290                 295                 300
   cct gta aaa tat gta atg gaa cat ggg aca aag att tac gga tct tac      960
   Pro Val Lys Tyr Val Met Glu His Gly Thr Lys Ile Tyr Gly Ser Tyr
   305                 310                 315                 320
   ggc cgc aag aaa cgt cgc cag cgt cgc cgt ggt gga tca cac cat cat     1008
   Gly Arg Lys Lys Arg Arg Gln Arg Arg Arg Gly Gly Ser His His His
                   325                 330                 335
   cat cac cac taa 1020
   His His His
<210> 2
<211> 339
<212> PRT
<213> Proteus vulgaris
<400> 2
   Met Ser His Pro Asp Leu Asn Lys Leu Leu Glu Leu Trp Pro His Ile
   1 5 10 15
   Gln Glu Tyr Gln Asp Leu Ala Leu Lys His Gly Ile Asn Asp Ile Phe
   20 25 30
   Gln Asp Asn Gly Gly Lys Leu Leu Gln Val Leu Leu Ile Thr Gly Leu
   35 40 45
   Thr Val Leu Pro Gly Arg Glu Gly Asn Asp Ala Val Asp Asn Ala Gly
   50 55 60
   Gln Glu Tyr Glu Leu Lys Ser Ile Asn Ile Asp Leu Thr Lys Gly Phe
   65 70 75 80
   Ser Thr His His His Met Asn Pro Val Ile Ile Ala Lys Tyr Arg Gln
   85 90 95
   Val Pro Trp Ile Phe Ala Ile Tyr Arg Gly Ile Ala Ile Glu Ala Ile
   100 105 110
   Tyr Arg Leu Glu Pro Lys Asp Leu Glu Phe Tyr Tyr Asp Lys Trp Glu
   115 120 125
   Arg Lys Trp Tyr Ser Asp Gly His Lys Asp Ile Asn Asn Pro Lys Ile
   130 135 140
   Pro Val Lys Tyr Val Met Glu His Gly Thr Lys Ile Tyr Gly Ser Gly
   145 150 155 160
   Gly Ser His Pro Asp Leu Asn Lys Leu Leu Glu Leu Trp Pro His Ile
   165 170 175
   Gln Glu Tyr Gln Asp Leu Ala Leu Lys His Gly Ile Asn Asp Ile Phe
   180 185 190
   Gln Asp Asn Gly Gly Lys Leu Leu Gln Val Leu Leu Ile Thr Gly Leu
   195 200 205
   Thr Val Leu Pro Gly Arg Glu Gly Asn Asp Ala Val Asp Asn Ala Gly
   210 215 220
   Gln Glu Tyr Glu Leu Lys Ser Ile Asn Ile Asp Leu Thr Lys Gly Phe
   225 230 235 240
   Ser Thr His His His Met Asn Pro Val Ile Ile Ala Lys Tyr Arg Gln
   245 250 255
   Val Pro Trp Ile Phe Ala Ile Tyr Arg Gly Ile Ala Ile Glu Ala Ile
   260 265 270
   Tyr Arg Leu Glu Pro Lys Asp Leu Glu Phe Tyr Tyr Asp Lys Trp Glu
   275 280 285
   Arg Lys Trp Tyr Ser Asp Gly His Lys Asp Ile Asn Asn Pro Lys Ile
   290 295 300
   Pro Val Lys Tyr Val Met Glu His Gly Thr Lys Ile Tyr Gly Ser Tyr
   305 310 315 320
   Gly Arg Lys Lys Arg Arg Gln Arg Arg Arg Gly Gly Ser His His His
   325 330 335
   His His His
<210> 3
<211> 48
<212> DNA
<213> Human immunodeficiency virus
<220>
<221> CDS
<222> (1)..(48)
<223> 4..36 Deliverer corresponding to the residues 47-57 of the protein Ta t
<400> 3
   gga tct tac ggc cgc aag aaa cgt cgc cag cgt cgc cgt ggt gga tca      48
   Gly Ser Tyr Gly Arg Lys Lys Arg Arg Gln Arg Arg Arg Gly Gly Ser
   1               5                   10                  15
<210> 4
<211> 16
<212> PRT
<213> Human immunodeficiency virus
<400> 4
   Gly Ser Tyr Gly Arg Lys Lys Arg Arg Gln Arg Arg Arg Gly Gly Ser
   1               5                   10                  15